# EUROPEAN PATENT SPECIFICATION

(11) **EP 1 206 474 B1**
(45) Date of publication and mention of the grant of the patent: **26.05.2004**
(21) Application number: 00955867.7
(22) Date of filing: 24.08.2000
(51) Int. Cl.: C07D 498/04, C07D 498/14, C07D 231/24, C07D 231/38, C07D 231/18, C07D 409/04, A61K 31/415, A61K 31/4155, A61K 31/4162, A61K 31/5365, A61K 31/424, A61K 31/55, A61P 19/02, A61P 29/00, A61P 35/00

(54) **SULFONYLPHENYLPYRAZOLE COMPOUNDS USEFUL AS COX-2 INHIBITORS**
ALS COX-HEMMER VERWENDBARE SULFONYLPHENYLPYRAZOL-VERBINDUNGEN
COMPOSES SULFONYLPHENYLPYRAZOLES UTILES EN TANT QU'INHIBITEURS DE COX-2

(30) Priority: 27.08.1999 US 384954
(43) Date of publication of application: 22.05.2002
(73) Proprietor: ABBOTT LABORATORIES, Abbott Park, IL 60064-6050 (US)
(72) Inventor: Kolasa, Teodozyj, Lake Villa, IL 60046 (US); Patel, Meena V., Green Oaks, IL 60048 (US)
(74) Representative: Modiano, Guido, Dr.-Ing.
(86) International application number: PCT/US2000/023214
(87) International publication number: WO 2001/016138

(56) References cited:
- EP-A- 0 414 200
- EP-A- 0 418 845
- EP-A- 0 531 901
- EP-A- 0 554 829
- WO-A-95/15317
- WO-A-96/14302
- WO-A-96/15115
- WO-A-97/13755
- US-A- 4 824 834
- K. TSUJI ET AL.: "Sudies on Anti-inflammatory Agents. IV. Synthesis and Pharmacological Properties of 1,5-Diarylpyrazoles and Related Derivatives" CHEMICAL & PHARMACEUTICAL BULLETIN, vol. 45, no. 6, 1997, pages 987-95, XP002153198
- PATENT ABSTRACTS OF JAPAN vol. 015, no. 388 (C-0872), 2 October 1991 (1991-10-02) & JP 03 157385 A (OTSUKA PHARMACEUT FACTORY INC), 5 July 1991 (1991-07-05)
- PATENT ABSTRACTS OF JAPAN vol. 1995, no. 08, 29 September 1995 (1995-09-29) & JP 07 118272 A (OTSUKA PHARMACEUT CO LTD), 9 May 1995 (1995-05-09)
- PATENT ABSTRACTS OF JAPAN vol. 1999, no. 02, 26 February 1999 (1999-02-26) & JP 10 310578 A (YOSHITOMI PHARMACEUT IND LTD), 24 November 1998 (1998-11-24)

## Description

### Technical Field

The present invention encompasses novel sulfonylphenylpyrazole compounds useful in the treatment of cyclooxygenase-2 mediated diseases. More particularly, this invention concerns a method of inhibiting prostaglandin biosynthesis, particularly the induced prostaglandin endoperoxide H synthase (PGHS-2, cyclooxygenase-2, COX-2) protein.

### Background of the Invention

The prostaglandins are extremely potent substances which produce a wide variety of biological effects, often in the nanomolar to picomolar concentration range. The discovery of two forms of prostaglandin endoperoxide H synthase that catalyze the oxidation of arachidonic acid leading to prostaglandin biosynthesis has resulted in renewed research to delineate the role of these two isozymes in physiology and pathophysiology. These isoenzymes PGHS-1 and PGHS-2 are more commonly referred to as cyclooxygenase-1 or COX-1 and cyclooxygenase-2 or COX-2. These isozymes have been shown to have different gene regulation and represent distinctly different prostaglandin biosynthesis pathways.

The PGHS-1 or COX-1 pathway is expressed constitutively in most cell types. This is an important "housekeeping" enzyme in many tissues, including the gastrointestinal (GI) tract and the kidneys. It responds to produce prostaglandins that regulate acute events in vascular homeostasis and also has a role in maintaining normal stomach and renal function. The PGHS-2 or COX-2 pathway involves an induction mechanism which has been linked to inflammation, mitogenesis and ovulation phenomena. COX-2 is the inducible isoform associated with inflammation.

Prostaglandin inhibitors provide therapy for pain, fever, and inflammation, and are useful therapies, for example in the treatment of rheumatoid arthritis and osteoarthritis. The non-steroidal anti-inflammatory drugs (NSAIDs) such as ibuprofen, naproxen and fenamates inhibit both isozymes. Inhibition of the constitutive enzyme PGHS-1 results in gastrointestinal side effects including ulcers and bleeding and incidence of renal problems with chronic therapy. Inhibitors of the induced isozyme PGHS-2 may provide anti-inflammatory activity without the side effects of PGHS-1 inhibitors.

The problem of side-effects associated with NSAID administration has never completely been solved in the past. Enteric coated tablets and co-administration with misoprostol, a prostaglandin derivative, have been tried in an attempt to minimize stomach toxicity. It would be advantageous to provide compounds which are selective inhibitors of the induced isozyme PGHS-2.

The present invention discloses novel compounds which are selective inhibitors of PGHS-2.

### Summary of the Invention

The present invention discloses sulfonylphenylpyrazole compounds which are selective inhibitors of cyclooxygenase-2 (COX-2).

The compounds of the present invention are selected from the group having the formula **I** below 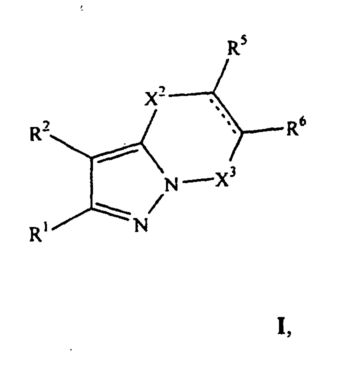 wherein
one of R¹ and R² is selected from the group consisting of: 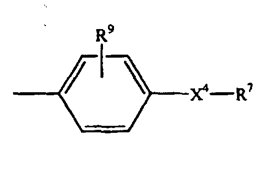 or 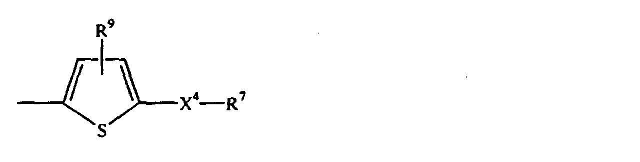 wherein
R⁷ is selected from the group consisting of alkyl, amino, alkylamino, dialkylamino;
X⁴ is selected from the group consisting of -SO₂-, -SO(NR⁸)-;
R⁸ is selected from the group consisting of hydrogen, alkyl, and cycloalkyl;
R⁹ is selected from the group consisting of hydrogen and halogen; and
the other of R¹ and R² is selected from the group consisting of hydroxyalkyl, halogen, alkyl, alkenyl, alkynyl, alkoxyalkyl, (NHR¹⁰)-C(O)-alkyl-, dimethylaminocarbonylalkyl, haloalkyl, cycloalkyl, cycloalkylalkyl, cycloalkenyl, cycloalkenylalkyl, aminocarbonylalkyl, aryl, heterocyclic, heterocyclic(alkyl), cyano, nitro, -Y-R¹⁰, - C(R¹¹)(R¹²)-halogen and -C(R¹¹)(R¹²)-OH;
Y is selected from the group consisting of -O-, -S-, - C(R¹¹)(R¹²)-, -C(O)NR¹⁴-, -C(O)-, -C(O)O-, -NH-, -NR¹⁴C(O)-, and - N(R¹³)-;
R¹⁰ is selected from the group consisting of hydrogen, alkyl, alkenyl, alkynyl, cycloalkyl, cycloalkenyl, amino, cyano, aryl, arylalkyl, heterocyclic, and heterocyclic(alkyl);
R¹¹, R¹² and R¹³ are independently selected from the group consisting of alkyl, alkenyl, cycloalkyl, cycloalkenyl, alkoxy, aryl, arylalkyl, heterocyclic, heterocyclic(alkyl) and cyano;
R¹⁴ is selected from the group consisting of hydrogen, alkyl, alkenyl, cycloalkyl, cycloalkenyl, alkoxy, aryl, arylalkyl, heterocyclic, heterocyclic(alkyl) and cyano;
X² is selected from the group consisting of -O-(CH₂)ₙ- and -S-(CH₂)ₙ-, wherein n is 0, 1 or 2;
X³ is absent, or is selected from the group consisting of -CH₂-and -C(R¹⁵)(R¹⁶)- wherein R¹⁵ and R¹⁶ are independently selected from the group consisting of hydrogen and alkyl;
R⁵ and R⁶ are selected from the group consisting of hydrogen, alkyl, substituted alkyl, alkenyl, alkynyl, aryl, cycloalkyl, heterocyclic, aryl(substituted alkyl) and arylalkyl, or R⁵ and R⁶ are taken together with the atoms to which they are attached to form a 5- to 7-membered ring, optionally aromatic, and optionally containing one or two heteroatoms selected from O, N and S, and optionally substituted with 1 to 2 groups selected from alkyl, hydroxy, halogen, oxo, haloalkyl, cyano and nitro;
provided that when n is 2, then X³ is absent and R⁵ and R⁶ taken together with the atom to which they are attached form a benzene ring;
the dashed bond represents an optional double bond;
aryl is a mono- or bicyclic carbocyclic ring system having one or two aromatic rings, wherein aryl by itself or as part of another group may be optionally substituted with one, two or three substituents selected from the group consisting of C₁-C₆ alkyl, haloalkyl, alkoxy, thioalkoxy, amino, alkylamino, dialkylamino, hydroxy, halo, mercapto, nitro, carboxaldehyde, carboxy, alkoxycarbonyl and carboxamide, or aryl may be tetrafluorophenyl or pentafluorophenyl;
heterocyclic is a 3- or 4-membered ring containing a heteroatom selected from oxygen, nitrogen and sulfur, or a 5-, 6- or 7-membered ring containing one, two or three nitrogen atoms, one nitrogen and one sulfur atom, or one nitrogen and one oxygen atom, wherein the 5-membered ring has 0-2 double bonds and the 6- and 7-membered rings have 0-3 double bonds and wherein the nitrogen atoms may be optionally quaternized, or a bicyclic group in which any of the above heterocyclic rings is fused to a benzene ring or cyclohexane ring or another heterocyclic ring as defined above, wherein heterocyclic by itself or as part of another group may be optionally substituted with one or two substituents independently selected from hydroxy, halo, oxo, C₁-C₆ alkylimino, amino, alkylamino, dialkylamino, alkoxy, alkoxyalkoxy, haloalkyl, cycloalkyl, aryl, arylalkyl, -COOH, -SO₃H and C₁-C₆ alkyl, or nitrogen-containing heterocyclics can be N-protected;
cycloalkyl is an aliphatic ring system having 3 to 10 carbon atoms and 1 to 3 rings, wherein cycloalkyl by itself or as part of another group may be optionally substituted with one, two or three substituents independently selected from C₁-C₆ alkyl, haloalkyl, alkoxy, thioalkoxy; amino, alkylamino, dialkylamino, hydroxy, halo, mercapto, nitro, carboxaldehyde, carboxy, alkoxycarbonyl and carboxamide;
cycloalkenyl is a cyclic hydrocarbon containing from 3 to 8 carbon atoms and at least one carbon-carbon double bond, wherein cycloalkenyl may be optionally substituted with one to five substituents independently selected from alkenyl, alkoxy, alkoxycarbonyl, alkoxycarbonylalkyl, alkyl, alkylcarbonyloxy, alkylcarbonyloxyalkyl, alkynyl, -C(O)-NHR¹⁰, dimethylaminocarbonyl, - alkyl-C(O)-NHR¹⁰, dimethylaminocarbonylalkyl, arylalkoxycarbonyl, arylalkoxycarbonylalkyl, arylcarbonyloxy, arylcarbonyloxyalkyl, aryloxycarbonyl, aryloxycarbonylalkyl, halo, haloalkoxy, haloalkyl, hydroxy, hydroxyalkyl, sulfamylalkyl, -NHR¹⁰ and (NHR¹⁰)alkyl;
substituted alkyl is a C₁-C₆ alkyl group substituted with 2, 3 or 4 substituents independently selected from the group consisting of alkoxy, alkoxycarbonyl, alkylcarbonyl, alkylcarbonyloxy, -C(O)-NHR¹⁰, dimethylaminocarbonyl, aryl, arylalkoxycarbonyl, arylcarbonyloxy, aryloxycarbonyl, -CF₃, cyano, cycloalkyl, halo, haloalkoxy, heterocycle, hydroxy, sulfamyl, alkylsulfonyl, arylsulfonyl and ―NHR¹⁰;
or a pharmaceutically acceptable salt, ester, or prodrug thereof.

### Detailed Description of the Invention

The present invention discloses sulfonylphenylpyrazole compounds which are cyclooxygenase (COX) inhibitors and are selective inhibitors of cyclooxygenase-2 (COX-2).

The compounds of the present invention are selected from the group having the formula **I** as defined above.

In another embodiment of the present invention compounds are selected from the group having the formula **I** wherein
R¹ is selected from the group consisting of: 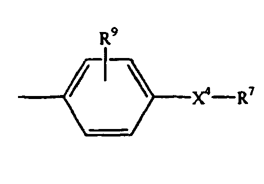 or

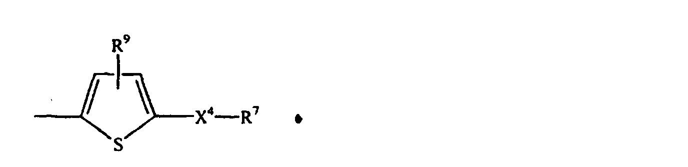

In another embodiment of the present invention are compounds having formula I wherein X² is oxygen, R¹ and R² are as defined above, X³ is absent or -CH₂-, and R⁵ and R⁶ form a 5 to 7 membered aromatic or non-aromatic carbocyclic ring, said carbocyclic ring optionally being mono, di, or trisubstituted with halogen.

Another embodiment of the present invention are compounds having formula **I** wherein X² is oxygen, R¹ and R² are as defined above, X³ is absent or -CH₂-, R⁵ and R⁶ are independently selected from the group consisting of hydrogen, alkyl, cyano, and aryl.

Preferred compounds of the present invention include:
3-(4-Fluorophenyl)-2-(4-(methylsulphonyl)phenyl)-5*H*-pyrazolo[1,5-*a*][3,1]-benzoxazine;
3-(4-Fluorophenyl)-2-(4-(methylsulphonyl)phenyl)-5,6,7,8-tetrahydropyrazoio[5,1-*b*][1,3]benzoxazole;
3-(*Tert*-butyl)-7-(4-fluorophenyl)-6-(4-(aminosulphonyl)phenyl)pyrazolo[5,1-*b*][1,3]-oxazole;
7-(4-Fluorophenyl)-3-methyl-6-(4-(aminosulphonyl)phenyl)pyrazolo[5,1-*b*][1,3]oxazole-2-carbonitrile;
3-Ethyl-7-(4-fluorophenyl)-6-(4-(methylsulphonyl)phenyl)pyrazolo[5,1-b][1,3]oxazole;
3-Ethyl-7-(4-fluorophenyl)-6-(4-(aminosulphonyl)phenyl)pyrazolo[5,1-b][1,3]oxazole;
6-Chloro-3-(4-fluorophenyl)-2-(4-(methylsulphonyl)phenyl)-5H-pyrazolo[1,5-a][3,1]benzoxazine;
3-(4-Fluorophenyl)-2-(4-aminosulphonyl)phenyl)-5H-pyrazolo[1,5-a][3,1]benzoxazine; and
2,6-Bis(4-fluorophenyl)-3-methyl-7-(4-(methylsulphonyl)phenyl)pyrazolo[5,1-b][1,3]oxazole;
or a pharmaceutically acceptable salt, ester, amide, or prodrug thereof.

### Abbreviations

Abbreviations which have been used in the embodiments, descriptions of the scheme and the examples that follow are:

The terms "loweralkyl" or "alkyl" as used herein refer to straight or branched chain alkyl radicals containing from 1 to 6 carbon atoms including, but not limited to, methyl, ethyl, n-propyl, iso-propyl, n-butyl, iso-butyl, sec-butyl, t-butyl, n-pentyl, 1-methylbutyl, 2,2-dimethylbutyl, 2-methylpentyl, 2,2-dimethylpropyl, n-hexyl and the like.

The term "alkylamino" as used herein refers to R₅₁NH- wherein R₅₁ is a loweralkyl group, for example, ethylamino, butylamino, and the like.

The term "dialkylamino" as used herein refers to R₅₆R₅₇N- wherein R₅₆ and R₅₇ are independently selected from loweralkyl, for example diethylamino, methyl propylamino, and the like.

The term "alkenyl" as used herein refers to a monovalent group derived from a hydrocarbon containing at least one carbon-carbon double bond by the removal of a single hydrogen atom. Alkenyl groups include, for example, vinyl (ethenyl), allyl (propenyl), butenyl, 1-methyl-2-buten-1-yl and the like.

The term "alkylene" denotes a divalent group derived from a straight or branched chain saturated hydrocarbon by the removal of two hydrogen atoms, for example methylene, 1,2-ethylene, 1,1-ethylene, 1,3-propylene, 2,2-dimethylpropylene, and the like.

The term "alkoxy" as used herein refers to R₄₁O- wherein R₄₁ is a loweralkyl group, as defined above. Examples of alkoxy include, but are not limited to, ethoxy, tert-butoxy, and the like.

The term "alkoxyalkyl" as used herein refers to an alkoxy group as previously defined appended to an alkyl group as previously defined. Examples of alkoxyalkyl include, but are not limited to, methoxymethyl, methoxyethyl, isopropoxymethyl and the like.

The term "alkylcarbonylalkyl" as used herein refers to R₆₂C(O)R₆₃- wherein R₆₂ is alkyl and R₆₃ is an alkyl radical.

The term "alkynyl," as used herein, refers to a straight or branched chain hydrocarbon group containing from 2 to 10 carbon atoms and containing at least one carbon-carbon triple bond. Representative examples of alkynyl include, but are not limited to, acetylenyl, 1-propynyl, 2-propynyl, 3-butynyl, 2-pentynyl, 1-butynyl, and the like.

Representative examples of a -C(O)-NHR¹⁰ group, as defined herein, include, but are not limited to, aminocarbonyl, ethylaminocarbonyl, benzylaminocarbonyl, and the like.

Representative examples of an -alkyl-C(O)-NHR¹⁰ group include, but are not limited to, aminocarbonylmethyl, 2-(ethylaminocarbonyl)ethyl, 3-(benzylaminocarbonyl)propyl, and the like.

The term "aminocarbonyl" as used herein refers to H₂N-C(O)-.

The term "aminocarbonylalkyl" as used herein refers to H₂N-C(O)R₆₄- wherein R₆₄ is alkyl as defined herein.

The term "aryl" as used herein refers to a mono- or bicyclic carbocyclic ring system having one or more aromatic rings including, but not limited to, phenyl, naphthyl, tetrahydronaphthyl, naphthyridinyl, indanyl, indenyl and the like. Aryl groups can be unsubstituted or substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide. In addition, substituted aryl groups include tetrafluorophenyl and pentafluorophenyl.

The term "arylalkyl" as used herein refers to an aryl group as previously defined, appended to a loweralkyl radical, for example, benzyl and the like.

The term "arylalkylamino" as used herein refers to R₅₅NH- wherein R₅₅ is an arylalkyl group, for example benzylamino and the like.

The term "arylamino" as used herein refers to R₅₃NH- wherein R₅₃ is an aryl group, for example, anilino, and the like.

The term "arylcarbonylalkyl" as used herein refers to R₅₄C(O)R₅₆- wherein R₅₄is an aryl group and R₅₆ is alkylene.

The term "cyano," as used herein, refers to a -CN group.

The term "cycloalkenyl," as used herein, refers to a cyclic hydrocarbon containing from 3 to 8 carbons and containing at least one carbon-carbon double bond formed by the removal of two hydrogens. Representative examples of cycloalkenyl include, but are not limited to, cyclohexene, 1-cyclohexen-2-yl, 3,3-dimethyl-1-cyclohexene, cyclopentene, cycloheptene, and the like.

The cycloalkenyl groups can be substituted with 1, 2, 3, 4, or 5 substituents independently selected from alkenyl, alkoxy, alkoxycarbonyl, alkoxycarbonylalkyl, alkyl, alkylcarbonyloxy, alkylcarbonyloxyalkyl, alkynyl, - C(O)-NHR¹⁰, dimethylaminocarbonyl, -alkyl-C(O)-NHR¹⁰, dimethylaminocarbonylalkyl, arylalkoxycarbonyl, arylalkoxycarbonylalkyl, arylcarbonyloxy, arylcaxbonyloxyalkyl, aryloxycarbonyl, aryloxycarbonylalkyl, halo, haloalkoxy, haloalkyl, hydroxy, hydroxyalkyl, sulfamylalkyl, -NHR¹⁰, (NHR¹⁰)alkyl.

The term "cycloalkenylalkyl," as used herein, refers to a cycloalkenyl group, as defined herein, appended to the parent molecular moiety through an alkyl group, as defined herein. Representative examples of cycloalkenylalkyl include, but are not limited to, (2,6,6-trimethyl-1-cyclohexen-1-yl)methyl, 1-cyclohexen-1-ylmethyl, 2-(2-cyclohepten-1-yl)ethyl, and the like.

The term "cycloalkyl" as used herein refers to an aliphatic ring system having 3 to 10 carbon atoms and 1 to 3 rings including, but not limited to, cyclopropyl, cyclopentyl, cyclohexyl, norbornyl, adamantyl, and the like. Cycloalkyl groups can be unsubstituted or substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide.

The term "cycloalkylalkyl" as used herein refers to a cycloalkyl group appended to a loweralkyl radical, including but not limited to cyclohexylmethyl.

The term "cycloalkylcarbonylalkyl" as used herein refers to a R₅₇C(O)R₅₈-wherein R₅₇ is a cycloalkyl group as defined herein and R₅₈ is alkylene.

The term "diarylamino" as used herein refers to R₆₀R₆₁N- wherein R₆₀ and R₆₁ are both aryl as defined herein.

The term "halogen" or "halo" as used herein refers to I, Br, Cl or F.

The term "haloalkyl" as used herein refers to a lower alkyl radical, as defined above, bearing at least one halogen substituent, for example, chloromethyl, fluoroethyl or trifluoromethyl and the like.

The terms "heterocyclic ring" or "heterocyclic" or "heterocycle" as used herein refers to any 3- or 4-membered ring containing a heteroatom selected from oxygen, nitrogen and sulfur; or a 5-, 6- or 7-membered ring containing one, two or three nitrogen atoms; one nitrogen and one sulfur atom; or one nitrogen and one oxygen atom. The 5-membered ring has 0-2 double bonds and the 6- and 7-membered ring have 0-3 double bonds. The nitrogen heteroatoms can be optionally quaternized. The term "heterocyclic" also includes bicyclic groups in which any of the above heterocyclic rings is fused to a benzene ring or a cyclohexane ring or another heterocyclic ring (for example, indolyl, quinolyl, isoquinolyl, tetrahydroquinolyl, benzofuryl or benzothienyl and the like). Heterocyclics include: azetidinyl, pyrrolyl, pyrrolinyl, pyrrolidinyl, pyrazolyl, pyrazolinyl, pyrazolidinyl, imidazolyl, imidazolinyl, imidazolidinyl, pyridyl, piperidinyl, homopiperidinyl, pyrazinyl, piperazinyl, pyrimidinyl, pyridazinyl, oxazolyl, oxazolidinyl, isoxazolyl, isoxazolidinyl, morpholinyl, thiazolyl, thiazolidinyl, isothiazolyl, isothiazolidinyl, indolyl, quinolinyl, isoquinolinyl, benzimidazolyl, benzothiazolyl, benzoxazolyl, furyl, thienyl and benzothienyl.

Heterocyclics can be unsubstituted or monosubstituted or disubstituted with substituents independently selected from hydroxy, halo, oxo (=O), alkylimino (R*N= wherein R* is a loweralkyl group), amino, alkylamino, dialkylamino, alkoxy, alkoxyalkoxy, haloalkyl, cycloalkyl, aryl, arylalkyl, -COOH, -SO₃H and loweralkyl. In addition, nitrogen containing heterocycles can be N-protected.

The term "heterocyclic(alkyl)" as used herein refers to a heterocyclic group as defined above appended to a loweralkyl radical as defined above.

The term "heterocycliccarbonylalkyl" as used herein refers to a heterocycle as defeined herein appended to the parent molecular moiety through a carbonylalkyl (Heterocycle-C(O)R₆₄-), wherein R₆₄ is alkylene.

The term "hydroxyalkyl," as used herein, refers to a hydroxy group, as defined herein, appended to the parent molecular moiety through an alkyl group, as defined herein. Representative examples of hydroxyalkyl include, but are not limited to, hydroxymethyl, 2-hydroxyethyl, 3-hydroxypropyl, 2-ethyl-4-hydroxyheptyl, 2-hydroxy-1,1-dimethylethyl, 3-hydroxy-1,1-dimethylpropyl, and the like.

The term "nitro" as used herein refers to -NO₂.

The term "substituted alkyl," as used herein, refers to an alkyl group, as defined herein, substituted with 2, 3, or 4 substituents selected from alkoxy, alkoxycarbonyl, alkylcarbonyl, alkylcarbonyloxy,
-C(O)-NHR¹⁰, dimethylaminocarbonyl, aryl, arylalkoxycarbonyl, arylcarbonyloxy, aryloxycarbonyl, -CF₃, cyano, cycloalkyl, halo, haloalkoxy, heterocycle, hydroxy, sulfamyl, alkylsulfonyl, arylsulfonyl, and -N H R¹⁰, as defined herein. Representative examples of substituted alkyl include, but are not limited to, 3-cyano-1,1-difluoropropyl, 1,1-dichloro-3-cyanopropyl, 1,1-bis(trifluoromethyl)-3-cyano-2-propyl, and the like.

### Preparation of the Comyounds of the Invention

The compounds and processes of the present invention will be better understood in connection with the following synthetic Schemes 1-3 which illustrate the methods by which the compounds of the invention may be prepared.

### Description of Schemes

Compounds of the invention having formula I may be prepared according to Scheme 1. For compounds of formula I wherein X³ is absent, X² is -O-(CH₂)n- or -S-(CH₂)ₙ- and n is 0, compounds 7 and 17 can be treated with an alpha-halogenated ketone in the presence of K₂CO₃ in DMF or DMSO at about 50 °C to provide 3-alkylated derivative 28. Refluxing of the solution of compound 28 in toluene-AcOH mixture in the presence of p-tolunesulphonic acid or pyridinium p-toluenesulphonate gives the desired compounds 29, which are compounds of formula **I**. Alternately, the compounds in which n is instead 1 may be prepared by reaction first with ortho-bromo- or chloro-benzyl bromide(or chloride) derivatives in the presence of K₂CO₃ in DMF at 50 °C to provide compounds 30. These compounds 30 may be reacted via intramolecullar Ullman cyclisation in the presence Cu in pyridine or CuI in DMF to afford the desired compounds 31, which are compounds of formula I. R¹⁷ is selected from hydrogen, alkyl, hydroxy, halogen, haloalkyl, cyano and nitro.

Alternately, as described in Scheme 2, to prepare compounds of formula I wherein X³ is absent, X² is -O-(CH₂)n- or -S-(CH₂)ₙ- and n is 2, compounds 7 and 17 can be treated with ortho-bromo- or chloro-phenylethyl bromide(or chloride) compounds in the presence of K₂CO₃ in DMF at about 80 °C to provide compounds 32. The cyclization of compounds 32 to gi ve compounds 33 requires strong acid such as polyphosphoric acid or methanesulphonic acid, for example. 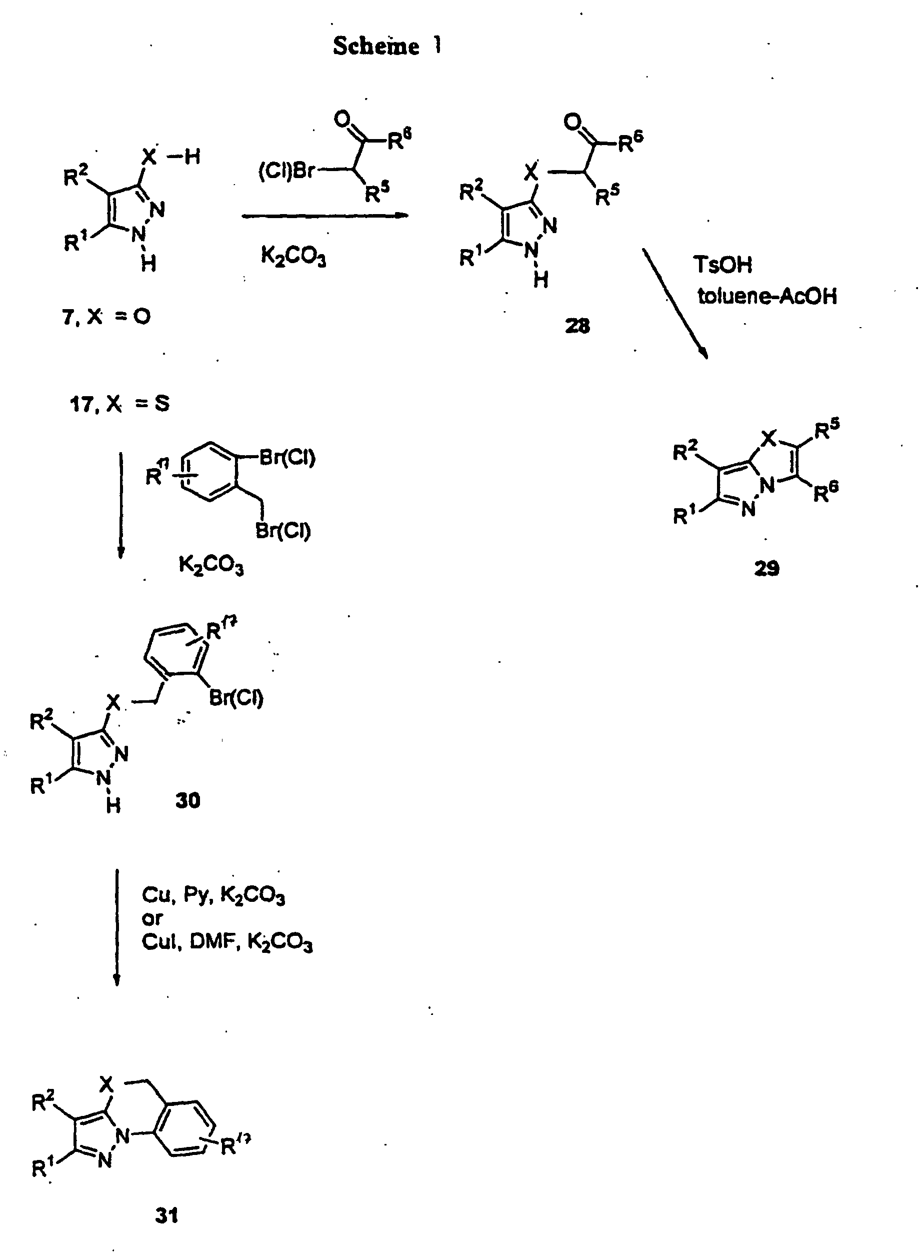 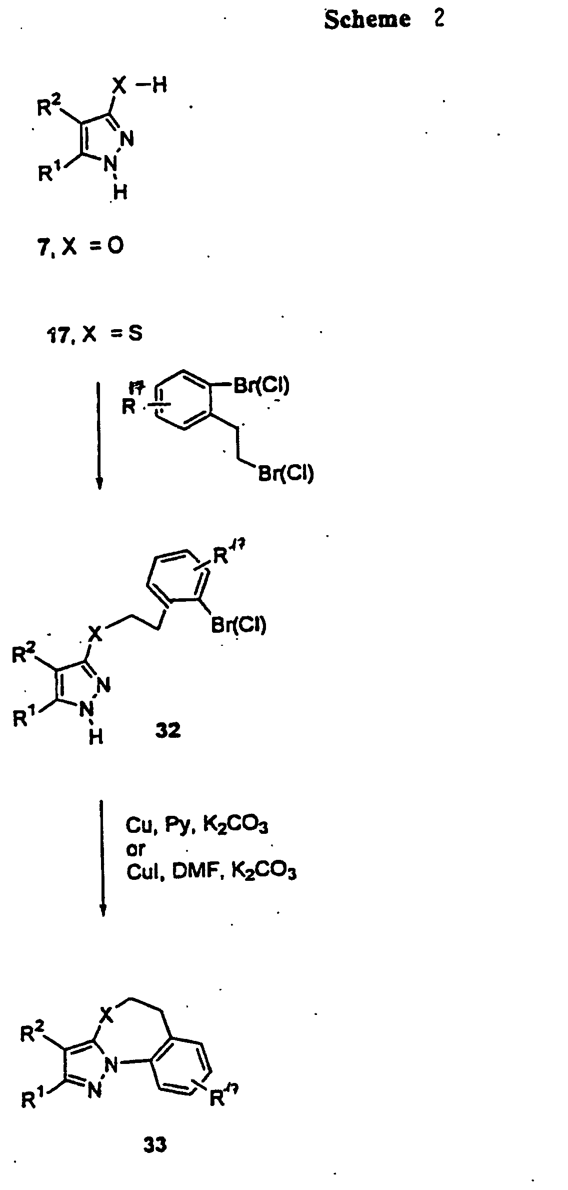 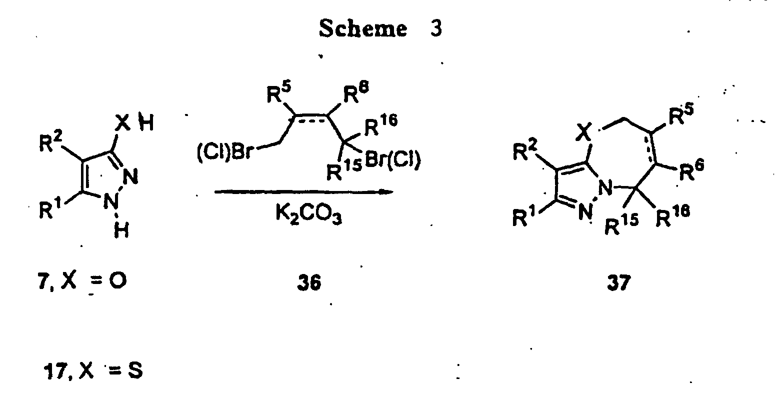 Compounds of the invention having formula I wherein X² is - O(CH₂)ₙ- or -S(CH₂)ₙ where n is 1
and X³ is C(R¹⁵)(R¹⁶) may be prepared according to Scheme 3. The compounds 7 and 17 can be treated at 50 °C with 1,4-dihalogene substituted compound 36 to give the desired compounds 37.

The compounds and processes of the present invention will be better understood in . connection with the following examples, which are intended as an illustration of and not a limitation upon the scope of the invention.

### Example 1

### 3-(4-Fluorophenyl)-2-(4-(methylsulphonyl) phenyl)-6,7-dihydro-5H-pyrazolo[5,1-b][1,3]oxazine

### 1A. Ethyl 2-(4-fluorophenyl)-3-(4-(methylthio)phenyl)-3-oxopropanoate

### 1A(i). 4-(Methylthio)benzoyl chloride and Ethyl 2-(4-fluorophenyl)acetate

A solution of 4-methylthiobenzoic acid (3.36 g, 20 mmol) and a few drops of DMF in anhydrous CH₂Cl₂ (50 mL) at 0 °C was treated dropwise with oxalyl chloride (4.4 mL, 50 mmol). The mixture was stirred at 0 °C for 6 hours. The reaction mixture was then concentrated *in vacuo* to provide crude 4-(methylthio)benzoyl chloride (yield: 3.7 g; ∼100%).

A mixture of 2-(4-fluorophenyl)acetic acid (10.8 g, 70 mmol) and concentrated H₂SO₄ (1 mL) in ethanol (150 mL) was refluxed for 8 hours. The reaction mixture was then concentrated *in vacuo*, and the residue was dissolved in ethyl ether. The ether solution was washed with 10% sodium bicarbonate, brine, dried over MgSO₄, and concentrated *in vacuo* to provide the ethyl ester (yield: 12.2 g; 96%).

### 1A(ii). Ethyl 2-(4-fluorophenyl)-3-(4-(methylthio)phenyl)-3-oxopropanoate

1 N Lithium bis(trimethylsilyl)amide (20 mL, 20 mmol) was added dropwise to a solution of ethyl (4-fluorophenyl)acetate prepared according to the method of Example 1A, (3.84 g, 20 mmol) in THF (20 mL) at -78 °C. After 15 minutes the mixture was treated dropwise with a suspension of crude 4-(methylthio)benzoyl chloride (3.7 g, 20 mmol) in THF (50 mL), and the resulting mixture was stirred at -78 °C for 60 minutes. The reaction was quenched with saturated NH₄Cl and extracted with ethyl acetate. The acetate layer was washed with water, brine, dried over anhydrous MgSO₄, and concentrated *in vacuo*. The residue was chromatographed (silica gel, 19:1 CH₂Cl₂₋ethyl acetate) to provide the desired product (yield: 4.55 g; 69%). MS (DCI-NH₃) m/z 333 (M+H)⁺, 350 (M+NH₄)⁺; ¹H NMR (300 MHz, CDCl₃) δ 1.25 (m, 3H), 2.50 (s, 3H), 4.20 (m, 2H), 5.53 (s, 1H), 7.03 (t, J = 9 Hz, 2H), 7.22 (d, J = 9 Hz, 2H), 7.38 (m, 2H), 7.85 (d, J = 9 Hz, 2H).

### 1B. Ethyl 2-(4-fluorophenyl)-3-(4-(methylthio)phenyl)-3-oxopropanoate

### 1B(i). 2-(4-Fluorophenyl)-1-(4-methoxyphenyl)ethan-1-one.

A suspension of magnesium turnings (2.4 g, 100 mmol) in anhydrous diethyl ether (200 mL) was prepared. A few drops of 1-(bromomethyl)-4-fluorobenzene were added and the mixture was warmed up to initiate the reaction. The remaining 1-(bromomethyl)-4-fluorobenzene (6.4 mL, 50 mmol), in diethyl ether (50 mL) was added slowly to maintain keep gentle boiling. Upon completion of addition the mixture was refluxed for 2 hours and cooled to 0 °C. The mixture was slowly transferred by canula to a solution of 4-methylthiobenzonitrile (7.46 g, 50 mmol). The reaction mixture was warmed to room temperature and stirred for 14 hours. The mixture was quenched with saturated NH₄Cl. The ethyl ether layer was washed with water, brine, dried over anhydrous MgSO₄, and concentrated *in vacuo*. The residue was purified by chromatography (silica gel, 5:4:1 hexanes-CH₂Cl₂-ethyl acetate) to provide the diaryl ketone (yield: 2.8 g; 22%). MS (DCI-NH₃) m/z 261 (M+H)⁺, 278 (M+NH₄)⁺; ¹H NMR (300 MHz, DMSO-d₆) δ 2.55 (s, 3H), 4.36 (s, 2H), 7.14 (t, J = 9 Hz, 2H), 7.29 (m, 2H), 7.38 (d, J = 9 Hz, 2H), 7.98 (d, J = 9 Hz, 2H).

### 1B(ii). Ethyl 2-(4-fluorophenyl)-3-(4-(methylthio)phenyl)-3-oxopropanoate

1 N Lithium bis(trimethylsilyl)amide (3.9 mL, 3.9 mmol) in anhydrous THF (10 mL) at -78 °C was treated dropwise with a solution of the diaryl ketone prepared according to the method of example 1 B(i) (1.03g, 3.9 mmol) in THF (25 mL). The mixture was stirred at -78 °C for 30 minutes and then ethyl cyanoformate (0.39 mL, 3.9 mmol) was added. The reaction mixture was stirred at -78 °C for 3 hours at room temperature for 3 hours. The mixture was quenched with saturated NH₄Cl and extracted with ethyl acetate. The ethyl acetate layer was washed with water, brine, dried over anhydrous MgSO₄, and concentrated *in vacuo*. The residue was purified by chromatography (silica gel, 5:4:1 hexanes-CH₂Cl₂₋ethyl acetate) to provide ethyl 2-(4-fluorophenyl)-3-(4-(methylthio)phenyl)-3-oxopropanoate (yield: 1.1 g; 83%). MS (DCI-NH₃) m/z 333 (M+H)⁺, 350 (M+NH₄)⁺; ¹H NMR (300 MHz, CDCl₃) δ 1.25 (m, 3H), 2.50 (s, 3H), 4.20 (m, 2H), 5.53 (s, 1 H), 7.03 (t, J = 9 Hz, 2H), 7.22 (d, J = 9 Hz, 2H), 7.38 (m, 2H), 7.85 (d, J = 9 Hz, 2H).

### 1C. 4-(4-Fluorophenyl)-5-(4-(methylthio)phenyl)-1H-pyrazol-3-ol.

A mixture of ethyl 2-(4-fluorophenyl)-3-(4-(methylthio)phenyl)-3-oxopropanoate(1.33 g, 4 mmol), prepared according to the method of Example 1A(ii), hydrazine hydrate (0.25 mL, 4.2 mmol), and acetic acid (0.25 mL, 4.2 mmol) in dioxane (50 mL), and water (5 mL) was refluxed for 24 hours and then concentrated *in vacuo*. Water was added to the residue, and the solid was filtered and dried *in vacuo* to provide the desired product (yield: 1.15 g; 95%). MS (DCI-NH₃) m/z 301 (M+H)⁺, 318 (M+NH₄)⁺; ¹H NMR (300 MHz, DMSO-d₆) δ 2.47 (s, 3H), 7.12 (t, J = 9 Hz, 2H), 7.26 (m, 6H).

### 1D. 3-(4-Fluorophenyl)-2-(4-(methylthio) phenyl)-6,7-dihydro-5H-pyrazolo[5,1-b][1,3]oxazine

A mixture of the 1*H*-pyrazol-3-ol, prepared according to the method of Example 1C, above (90 mg, 0.3 mmol), 1,3-dibromopropane (0.21 mL, 0.4 mmol) and anhydrous K₂CO₃ (1.1 g, 0.8 mmol) in DMF (40 mL) was refluxed at 50 °C for 7 hours. The mixture was poured into water and extracted with ethyl acetate. The organic solvent was removed *in vacuo* and the residue was purified by chromatography (silica gel, ethyl acetate) to provide the desired product (yield: 60 mg; 60%). MS (DCI-NH₃) m/z 341 (M+H)⁺.

### 1 E. 3-(4-Fluorophenyl)-2-(4-(methylsulphonyl) phenyl)-6,7-dihydro-5H-pyrazolo[5,1-b][1,3]oxazine

A solution of the 4-(methylthio)phenyl derivative, prepared according to the method of Example 1D, above (55 mg, 0.16 mmol) in CH₂Cl₂ (10 mL) at 0 °C was treated with 32% peracetic acid (0.3 mL), and the mixture was stirred at 0 °C for 75 minutes. The mixture was then washed with water, saturated sodium bicarbonate, brine, dried over anhydrous MgSO₄, and concentrated *in vacuo*. The residue was purified by chromatography (silica gel, ethyl acetate) to provide the desired product (yield: 35 mg; 59%). MP 198-200 °C; MS (DCI-NH₃) m/z 373 (M+H)⁺, 390 (M+NH₄)⁺; ¹H NMR (300 MHz, DMSO-d₆) δ 2.27 (m, 2H), 3.23 (s, 3H), 4.21 (t, J = 7 Hz, 2H), 4.38 (t, J = 7 Hz, 2H), 7.20 (m, 4H), 7.60 (d, J = 9 Hz, 2H), 7.86 (d, J = 9 Hz, 2H); Anal. calc. for C₁₉H₁₇FN₂O₃S•0.5 H₂O: C, 59.83; H, 4.75; N, 7.34. Found: C, 60.17; H, 4.72; N, 7.22.

### 1E(i). 3-(4-Fluorophenyl)-2-(4-(methylsulphonyl) phenyl)-6,7-dihydro-5H-pyrazolo[5,1-b][1.3]oxazine

Alternatively, the desired compound was prepared according to the method of Example 5D, starting with 4-(4-fluorophenyl)-3-hydroxy-5-(4-(methylsulphonyl)phenyl)-pyrazole and substituting 1,3-dibromopropane in place of 1,2-dibromo ethane (yield: 150 mg, 81%). MS (APCI+) m/z 373 (M+H)⁺; (APCI-) m/z 371 (M-H)⁻; ¹H NMR (300 MHz, DMSO-d₆) δ 2.27 (m, 2H), 3.23 (s, 3H), 4.20 (t, J = 7 Hz, 2H), 4.38 (d, J = 7 Hz, 2H), 7.20 (m, 4H), 7.60 (d, J = 9 Hz, 2H), 7.87 (d, J = 9 Hz, 2H); Anal. calc. for C₁₉H₁₇FN₂O₃S: C, 61.27; H, 4.60; N, 7.52. Found: C, 60.93; H, 4.48; N, 7.34.

### Example 2

### 2A. 3-(4-Fluorophenyl)-2-(4-(methylsulphonyl) phenyl)-5,6,7,8-tetrahydropyrazolo[5,1-b][1,3]oxazepine

The desired product was prepared according to the method of Example 1, substituting 1,4-dibromobutane in place of 1,3-dibromopropane (yield: 145 mg, 75%). MP 167-168 °C. MS (DCI-NH₃) m/z 387 (M+H)⁺; ¹H NMR (300 MHz, CDCl₃) δ 1.98 (m, 2H), 2.13 (m, 2H), 3.07 (s, 3H), 4.12 (t, J = 7 Hz, 2H), 4.26 (t, J = 7 Hz, 2H), 7.05 (t, J = 9 Hz, 2H), 7.20 (m, 2H), 7.65 (d, J = 9 Hz, 2H), 7.86 (d, J = 9 Hz, 2H); Anal. calc. for C₂₀H₁₉FN₂O₃S•0.25 H₂O: C, 61.44; H, 5.02; N, 7.16. Found: C, 61.64; H, 5.01; N, 7.08.

### 2B. 3-(4-Fluorophenyl)-2-(4-(methylsulphonyl) phenyl)-5,6,7,8-tetrahydropyrazolo[5,1-b][1,3]oxazepine

Alternatively, the desired product was prepared according to the method of Example 5D substituting 1,4-dibromobutane in place of 1,2-dibromoethane (yield: 145 mg, 75%). MS (DCI-NH₃) m/z 387 (M+H)⁺; ¹H NMR (300 MHz, DMSO-d₆) δ 1,86 (m, 2H), 2.03 (m, 2H), 3.22 (s, 3H), 4.12 (t, J = 7 Hz, 2H), 4.30 (t, J = 7 Hz, 2H), 7.22 (m, 4H), 7.58 (d, J = 9 Hz, 2H), 7.85 (d, J = 9 Hz, 2H); Anal. calc. for C₂₀H₁₉FN₂O₃S: C, 62.16; H, 4.95; N, 7.24. Found: C, 61.87; H, 5.11; N, 7.09.

### Example 3

### 3-(4-Fluorophenyl)-2-(4-methylsulphonyl) phenyl)-5,10-dihydropyrazolo[5,1-c][1,3]benzoxazepine

The desired product was prepared as described in Example 1, substituting 1,2-dibromomethylbenzene in place of 1,3-dibromopropane (yield: 55 mg, 26%). MS (DCI-NH₃) m/z 435 (M+H)⁺; ¹H NMR (300 MHz, CDCl₃) δ 3.04 (s, 3H), 5.35 (s, 2H), 5.63 (s, 2H), 7.01 (t, J = 9 Hz, 2H), 7.19 (m, 2H), 7.43 (m, 4H), 7.63 (d, J = 9 Hz, 2H), 7.85 (d, J = 9 Hz, 2H); Anal. calc. for C₂₄H₁₉FN₂O₃S•1.5 H₂O: C, 62.46; H, 4.80; N, 6.06. Found: C, 62.55; H, 4.39; N, 5.72.

### Example 4

### 3-(4-Fluorophenyl)-2-(4-methylsulponylphenyl) -5,8-dihydropyrazolo[5,1-b][1,3]oxazepine

The desired compound was prepared according to the method of Example 1, substituting 1,4-dibromo-2-butene in place of 1,3-dibromopropane (yield: 35 mg, 17%). MS (APCI+) m/z 385 (M+H)⁺; (APCI-) m/z 383 (M-H)⁻, 419 (M+Cl)⁻; ¹H NMR (300 MHz, CDCl₃) δ 3.11 (s, 3H), 4.20 (m, 2H), 4.90 (m, 2H), 5.60 (m, 1 H), 6.10 (m, 1 H), 6.92 (t J = 9 Hz, 2H), 7.12 (m, 2H), 7.47 (d, J = 9 Hz, 2H), 7.98 (d, J = 9 Hz, 2H); Anal. calc. for C₂₀H₁₇FN₂O₃S•0.25 H₂O: C, 61.76; H, 4.53; N, 7.20. Found: C, 61.70; H, 4.75; N, 6.56.

### Example 5

### 7-(4-Fluorophenyl)-6-(4-(methylsulphonyl) phenyl)-2,3-dihydropyrazolo[5,1-b][1,3]oxazole

### 5A. Methyl 2-(4-fluorophenyl)-3-(4-(methylsulphonyl)phenyl)-3-oxopropanoate.

A solution of methyl 2-(4-fluorophenyl)acetate (2.35 g, 14 mmol) in THF (15 mL), at -78 °C, was treated dropwise with 1 N lithium bis(trimethylsilyl)amide (14 mL, 14 mmol). After 15 minutes a suspension of 4-(methylsulphonyl)benzoyl chloride (3.3 g, 15 mmol) in THF (25 mL) was added in portions. The reaction mixture was stirred for 60 minutes at -78 °C and at 0 to 5 °C for 12 hours. The mixture was quenched with 10% citric acid, the THF removed *in vacuo*, and the residue triturated with hexanes to provide the desired product as a solid (yield: 3.4 g; 69%). MS (DCI-NH₃) m/z 368 (M+H)⁺; ¹H NMR (300 MHz, DMSO-d₆) δ 3.27 (s, 3H), 3.69 (s, 3H), 6.35 (s, 1H), 7.21 (m, 2H), 7.44 (m, 2H), 8.06 (d, J = 9 Hz, 2H), 8.25 (d, J = 9 Hz, 2H).

### 5B. Ethyl 2-(4-fluorophenyl)-3-(4-(methylsulphonyl)phenyl)-3-oxopropanoate.

The desired compound was prepared according to the method of Example 5A, substituting ethyl 2-(4-fluorophenyl)acetate in place of methyl 2-(4-fluorophenyl)acetate (yield: 4.55 g, 69%).

### 5C. 4-(4-Fluorophenyl)-5-(4-methylsulphonylpheny)-1H-pyrazol-3-ol.

A mixture of the ethyl ester (2.09 g, 5.74 mmol), prepared according to the method of Example 5B, hydrazine hydrate (0.36 mL, 6 mmol), and acetic acid (0.36 mL, 6 mmol) in dioxane (100 mL), and water (10 mL) was heated at reflux for 24 hours. The dioxane was removed *in vacuo*. The residue was washed with water (50 mL), and the solid filtered and dried *in vacuo* to provide the desired product (yield: 1.8 g; 95%). MS (DCI-NH₃) m/z 333 (M+H)⁺, 350 (M+NH₄)⁺; ¹H NMR (300 MHz, DMSO-d₆) δ 3.26 (s, 3H), 7.17 (m, 2H), 7.27 (m, 2H), 7.57 (m, 2H), 7.93 (d, J = 9 Hz, 2H).

### 5D. 7-(4-Fluorophenyl)-6-(4-(methylsulphonyl) phenyl)-2,3-dihydropyrazolo[5,1-b][1,3]oxazole

The 1*H*-pyrazol-3-ol (83 mg, 0.25 mmol), prepared according to the method of Example 5C, K₂CO₃ (138 mg, I mmol) and 1,2-dibromoethane in DMF (30 mL) were refluxed at 50 °C for 8 hours. The mixture was partitioned between water and ethyl acetate. The acetate layer was washed with water, brine, dried with anhydrous MgSO₄, and concentrated *in vacuo*. The residue was chromatographed (silica gel, ethyl acetate) to provide the desired product (yield: 80 mg; 92%). MS (DCI-NH₃) m/z 359 (M+H)⁺, 376 (M+NH₄)⁺; ¹H NMR (300 MHz, DMSO-d₆) δ 3.03 (s, 3H), 4.42 (t, J = 7 Hz, 2H), 5.20 (t, J = 7 Hz, 2H), 7.20 (d, J = 9 Hz, 4H), 7.63 (d, J = 9 Hz, 2H), 7.90 (d, J = 9 Hz, 2H); Anal. calc. for C₁₈H₁₅FN₂O₃S•0.25 H₂O: C, 59.57; H, 4.30; N, 7.71. Found: C, 59.33; H, 4.35; N, 7.67.

### Example 6

### 3-(4-Fluorophenyl)-2-(4-(methylsulphonyl)phenyl)-5H-pyrazolo[1,5-b][1,3]-benzoxazine

### 6A. 3-(4-Fluorophenyl)-2-(4-(methylsulphonyl) phenyl)-5H-pyrazolo[1,5-b][1,3]-benzoxazine

A mixture of the 1*H*-pyrazol-3-ol (133 mg, 0.4 mmol), prepared according to the method of Example 5C, and anhydrous K₂CO₃ (69 mg, 0.5 mmol) in pyridine (25 mL) were treated with 1-bromo-2-(bromomethyl)benzene (100 mg, 0.4 mmol) and copper powder (20 mg). The reaction mixture was stirred at room temperature for 14 hours. The mixture was then poured into 10% citric acid and extracted with ethyl acetate. The acetate layer was washed with water, brine, dried over anhydrous MgSO₄, and concentrated *in vacuo*. The residue was chromatographed (silica gel, 3:2 hexanes-ethyl acetate) to provide the desired product (yield: 12 mg; 8%). ¹H NMR (300 MHz, DMSO-d₆) δ 3.24 (s, 3H), 5.52 (s, 2H), 7.30 (m, 5H), 7.43 (d, J = 8 Hz, 1H), 7.53 (m, 1H), 7.74 (t, J = 9 Hz, 3H), 7.96 (d, J = 9 Hz, 2H); MS (APCI+Q1) m/z 421 (M+H)⁺; (APCI - Q1) m/z 420 (M)⁺. Anal. calc. for C₂₃H₁₇FN₂O₃S: C, 65.70; H, 4.07; N, 6.66. Found: C, 65.08; H, 3.99; N, 6.47.

### 6B. 3-(4-Fluorophenyl)-2-(4-(methylsulphonyl) phenyl)-5H-pyrazolo[1,5-b][1,3]-benzoxazine

A solution of the 1*H*-pyrazol-3-ol derivative (200 mg, 0.6 mmol), prepared according to the method of Example 5C, and K₂CO₃ (97 mg, 0.7 mmol) in DMF (25 mL) at 50 °C was treated dropwise with a solution of 1-bromo-2-(bromomethyl)benzene (175 mg, 0.7 mmol) in DMF (5 mL). The mixture was stirred until the starting material disappeared (∼40 minutes). The mixture was treated with K₂CO₃ (200 mg, 1.5 mmol) and copper (I) iodide (CuI, 30 mg), heated at 150 °C until the starting material disappeared, approximately 2 hours. The reaction mixture was then cooled to room temperature, poured into 10% citric acid, and extracted with ethyl acetate. The acetate extract was concentrated *in vacuo*, and the residue was purified to provide the desired product (yield: 150 mg; 60%). MS (APCI+) m/z 421 (M+H)⁺; (APCI -) m/z 420 (M)⁺; ¹H NMR (300 MHz, DMSO-d₆) δ 3.26 (s, 3H), 5.51 (s, 2H), 7.30 (m, 5H), 7.43 (d, J = 8 Hz, 1H), 7.53 (m, 1H), 7.74 (t, J = 9 Hz, 3H), 7.95 (d, J = 9 Hz, 2H); Anal. calc. for C₂₃H₁₇FN₂O₃S•0.5 H₂O: C, 64.32; H, 4.22; N, 6.59. Found: C, 64.25; H, 4.26; N, 6.25.

### 6C. 4-(4-Fluorophenyl)-5-(4-(methylsulphonyl) phenyl)-3-(2-bromobenzyloxy)-1-(2-bromobenzyl)-1H-pyrazole

The desired product was isolated from the reaction mixture of Example 6B, by chromatography (yield: 85 mg, 21%). MS (APCI+) m/z 671 (M+H)⁺; (APCI-) m/z 705 (M+Cl)⁻; ¹H NMR (300 MHz, DMSO-d₆) δ 3.25(s, 3H), 5.18 (s, 2H), 5.34 (s, 2H), 6.86 (m, 1H), 7.10 (t, J = 9 Hz, 2H), 7.20 (m, 2H), 7.32 (m, 2H), 7.40 (m, 1H), 7.55 (d, J = 9 Hz, 3H), 7.66 (d, J = 9 Hz, 1H), 7.95 (d, J = 9 Hz, 2H); Anal. calc. for C₃₀H₂₃Br₂FN₂O₃S•0.5 H₂O: C, 53.03; H, 3.56; N, 4.12. Found: C, 53.12; H, 3.66; N, 3.74.

### Example 20A

### 2-{4-(4-Fluorophenyl)-5-(4-(methylsulphonyl)phenyl)-3-[2-(2-thienyl)-2-oxoethoxy]-1H-pyrazol-1-yl}-1-(2-thienyl)ethan-1-one

A mixture of 4-(4-fluorophenyl)-5-(4-methylsulphonylpheny)-1*H*-pyrazol-3-ol prepared according to the method of Example 5C (332 mg, I mmol) and K₂CO₃ (138 mg, 1 mmol) in DMF (25 mL) at 40 °C was treated dropwise with a solution of bromomethyl 2-thienyl ketone (204 mg, 1 mmol) in DMF (5 mL). The resulting mixture was stirred at 40 °C for 1 hour, then poured into icy 10% citric acid, and extracted with ethyl acetate. The acetate extract was washed with water, brine, dried over MgSO₄, and concentrated *in vacuo*. The residue was chromatographed (silica gel, 1:1 hexanes-ethyl acetate, ethyl acetate) to provide the desired product (yield: 50 mg; 9%). MP 136-138 °C; MS (APCI+) m/z 581 (M+H)⁺; (APCI-) m/z 579 (M-H)⁻, 615 (M+Cl)⁻; ¹H NMR (300 MHz, DMSO-d₆) δ 3.22 (s, 3H), 5.45 (s, 2H), 5.62 (s, 2H), 7.14 (m, 2H), 7.26 (m, 4H), 7.55 (d, J = 9 Hz, 2H), 7.95 (m, 3H), 8.06 (m, 3H); Anal calc. for C₂₈H₂₁FN₂O₅S₃•0.5 H₂O: C, 57.03; H, 3.76; N, 4.75. Found: C, 56.98; H, 3.82; N, 4.70.

### Example 20B

### 2-[(4-(4-Fluorophenyl)-5-(4-(methylsulphonyl)phenyl)-1H-pyrazol-3-yl)oxy]-1-(2-thienyl)ethan-1-one.

The mono-alkylated product was isolated from the reaction mixture prepared in Example 20A by chromatography (silica gel, 1:1 hexanes-ethyl acetate, ethyl acetate)(yield: 270 mg; 60%).

### Example 23

### 3-[(1-Ethyl-4-(4-fluorophenyl)-5-(4-(methylsulphonyl)phenyl)-1H-pyrazol-3-yl)oxy]tetrahydro-4H-pyran-4-one

### 23A. 3-Bromo-tetrahydro-4H-pyran-4-one.

To a solution of tetrahydro-4H-pyran-4-one (100 mg, 1 mmol) in THF (30 mL) was added dropwise a solution of pyrrolidone hydrotribromide (496 mg, 1 mmol) in THF (10 mL). The mixture was stirred at room temperature for 2 hours and concentrated *in vacuo*. Ethyl acetate and water were added to the residue. The acetate layer was separated, washed with water, 10% NaHCO₃, brine and dried over MgSO₄. The ethyl acetate was removed *in vacuo* to provide crude 3-bromo-tetrahydro-4H-pyran-4-one (yield: 150 mg, 84%).

### 23B. 3-[(4-(4-Fluorophenyl)-5-(4-(methylsulphonyl)phenyl)-1H-pyrazol-3-yl)oxy]tetrahydro-4H-pyran-4-one

A mixture of 1*H*-pyrazol-3-ol prepared according to the method of Example 5C (235 mg, 0.7 mmol) and K₂CO₃ (96 mg, 0.7 mmol) in DMF (25 mL) was prepared and added to the 3-bromo-tetrahydro-4H-pyran-4-one compound (150 mg, 0.84 mmol), prepared according to the method of Example 23A. The resulting mixture was stirred at room temperature for 14 hours. Citric acid (10%) was added and the mixture extracted with ethyl acetate. The acetate layer was washed with water, brine, dried over MgSO₄ and concentrated *in vacuo* to provide the crude pyrazole (yield: 400 mg, 110%).

### 23C. 1-Ethyl-4-(4-fluorophenyl)-5-(4-(methylsulphonyl)phenyl)-3-(tetrahydro-4H-pyran-4-on-3-yloxy)pyrazole.

A mixture of the pyrazole derivative (43 mg, 0.1 mmol), prepared according to the method of Example 23B, K₂CO₃ (28 mg, 0.2 mmol) and iodoethane (0.02 mL, 0.2 mmol) in acetone (10 mL) was refluxed for 6 hours. The mixture was concentrated *in vacuo* and chromatographed (silica gel, ethyl acetate) to provide the desired product (yield: 20 mg, 48%). MP 181-182 °C; MS (APCI+) m/z 459 (M+H)⁺; (APCI-) m/z 493 (M+Cl)⁻;¹H NMR (300 MHz, DMSO-d₆) δ 1.12 (t, J = 7 Hz, 3H), 2.44 (m, 1H), 2.90 (m, 1H), 3.30 (s, 3H), 3.61 (t, J = 12 Hz, 2H), 3.83 (q, J = 7 Hz, 2H), 4.18 (m, 1H), 4.47 (m, 1 H), 5.40 (m, 1H), 7.13 (m, 4H), 7.62 (d, J = 9 Hz, 2H), 8.01 (d, J = 9 Hz, 2H).

### Example 25

### 1-[3-(3,3-Dimethyl-2-oxobutoxy)-4-(4-fluorophenyl)-5-(4-(methylsulphonyl)phenyl)-1H-pyrazol-1-yl]-3,3-dimethylbutan-2-one

The desired compound was prepared according to the method of Example 20, substituting bromopinacolone in place of bromomethyl 2-thienyl ketone. (yield: 140 mg, 44%). MP 180-182 °C; MS (APCI+) m/z 529 (M+H)⁺; (APCI-) m/z 527 (M-H)⁻, 563 (M+Cl)⁻; ¹H NMR (300 MHz, DMSO-d₆) δ 0.95 (s, 9H), 1.14 (s, 9H), 3.23 (s, 3H), 5.00 (s, 2H), 5.22 (s, 2H), 7.10 (m, 2H), 7.22 (m, 2H), 7.49 (d, J = 9 Hz, 2H), 7.97 (d, J = 9 Hz, 2H); Anal. calc. for C₂₈H₃₃FN₂O₅S•0.25 H₂O: C, 63.08; H, 6.33; N, 5.25. Found: C, 62.95; H, 6.39; N, 5.14.

### EXAMPLE 32

### 1-[(1-Acetyl-5-(4-(methylsulphonyl)phenyl)-4-phenyl-1H-pyrazol-3-yl)oxy]-3,3-dimethylbutan-2-one

### 32A. 5-(4-(Methylsulphonyl)phenyl)-4-phenyl-1H-pyrazol-3-ol

The desired compound was prepared according to the method of Example 5A, substituting ethyl 2-phenylacetate in place of methyl 2-(4-fluorophenyl)acetate (yield: 3.12 g, 99%). MS (APCI+) m/z 315 (M+H)⁺; (APCI-) m/z 313 (M-H)⁻, 349 (M+Cl)⁻; ¹H NMR (300 MHz, DMSO-d₆) δ 3.22 (s, 3H), 7.25 (m, 5H), 7.57 (d, J = 9 Hz, 2H), 7.89 (d, J = 9 Hz, 2H).

### 32B. 3,3-Dimethyl-1-[(5-(4-(methylsulphonyl) phenyl)-4-phenyl-1H-pyrazol-3-yl)oxy]butan-2-one.

The desired compound was prepared according to the method of Example 25 substituting 5-(4-(methylsulphonyl)phenyl)-4-phenyl-3-hydroxypyrazole in place of 4-(4-fluorophenyl)-5-(4-(methylsulphonyl)phenyl)-3-hydroxypyrazole. MS (APCI+) m/z 413 (M+H)⁺; (APCI-) m/z 411 (M-H)⁻, 447 (M+Cl)⁻; ¹H NMR (300 MHz, DMSO-d₆) δ 1.17 (s, 9H), 3.22 (s, 3H), 5.24 (s, 2H), 7.33 (m, 5H), 7.58 (d, J = 9 Hz, 2H), 7.94 (d, J = 9 Hz, 2H).

### 32C. 1-[(1-Acetyl-5-(4-(methylsulphonyl)phenyl)-4-phenyl-1H-pyrazol-3-yl)oxy]-3,3-dimethylbutan-2-one.

A mixture of 3,3-dimethyl-1-[(5-(4-(methylsulphonyl)phenyl)-4-phenyl-1*H*-pyrazol-3-yl)oxy]butan-2-one (145 mg, 0.35 mmol), prepared according to the method of Example 32B, p-toluene-sulfonic acid hydrate (15 mg), and molecular sieves (4 g) in toluene (35 mL) and acetic acid (5 mL) was refluxed for 24 hours. The mixture was concentrated *in vacuo*, and the residue was purified by chromatography (silica gel, 1:1 hexanes-ethyl acetate) to provide the desired product (yield: 30 mg; 19%). MP 189-191 °C; MS (APCI+) m/z 455 (M+H)⁺; ¹H NMR (300 MHz, DMSO-d₆) δ 1.22 (s, 9H), 2.48 (s, 3H), 3.28 (s, 3H), 5.37 (s, 2H), 7.23 (m, 5H), 7.60 (d, J = 9 Hz, 2H), 7.90 (d, J = 9 Hz, 2H); Anal. calc. for C₂₄H₂₆N₂O₅S•0.5 H₂O: C, 62.18; H, 5.87; N, 6.04. Found: C, 62.38; H, 5.81; N, 5.83.

### Example 36A

### 1-(4-Fluorophenyl)-2-{[4-(4-fluorophenyl)-5-(4-(methylsulphonyl)phenyl)-1H-pyrazol-3-yl]oxy}ethan-1-one

A mixture of 1*H*-pyrazol-3-ol derivative prepared according to the method of Example 5C, (200 mg, 0.6 mmol) and K₂CO₃ (83 mg, 0.6 mmol) in DMF (30 mL) at 50 °C was treated dropwise with a solution of 4'-fluoro-2-bromoacetophenone (130 mg, 0.6 mmol in DMF (10 mL), and stirred at 50 °C for 50 minutes. The mixture was then poured into water and extracted with ethyl acetate. The acetate layer was washed with water, brine, dried over anhydrous MgSO₄, and concentrated *in vacuo* to provide the desired product (yield: 280 mg; 99%). MS (APCI+) m/z 469 (M+H)⁺; (APCI-) m/z 467 (M-H)⁺; ¹H NMR (300 MHz, DMSO-d₆) δ 3.23 (s, 3H), 5.64 (s, 2H), 7.22 (m, 2H), 7.39 (m, 4H), 7.60 (d, J = 9 Hz, 2H), 7.95 (d, J = 9 Hz, 2H), 8.12 (m, 2H), 12.60 (s, 1H); Anal. calc. for C₂₄H₁₈F₂N₂O₄S: C, 61.53; H, 3.87; N, 5.97. Found: C, 61.28; H, 4.28; N, 5.45.

### Example 36B

### 3,7-Bis(4-fluorophenyl)-6-(4-(methylsulphonyl)phenyl)pyrazolo[5,1-b][1,3]oxazole

A mixture of the O-alkylated derivative from the Example 36A (47 mg, 0.1 mmol), p-toluenesulfonic acid hydrate (19 mg, 0.1 mmol) in toluene (20 mL) and acetic acid (7 mL) was refluxed for 4 hours using a Dean-Stark trap to remove water. The mixture was then concentrated *in vacuo*, and the residue was dissolved in ethyl acetate. The acetate solution was washed with sodium bicarbonate (10%), brine, dried over MgSO₄, and concentrated *in vacuo*. The residue was chromatographed (silica gel, 1:1 hexanes-ethyl acetate) to provide the desired product (yield: 40 mg; 89%). MP 200-202 °C; MS (APCI+) m/z 451 (M+H)⁺; (APCI-) m/z 449 (M-H)⁻, 485 (M+Cl)⁻; ¹H NMR (300 MHz, DMSO-d₆) δ 3.25 (s, 3H), 7.26 (t, J = 9 Hz, 2H), 7.45 (m, 4H), 7.80 (d, J = 9 Hz, 2H), 8.00 (d, J = 9 Hz, 2H), 8.30 (m, 2H), 8.83 (s, 1H); Anal. calc. for C₂₄H₁₆F₂N₂O₃S•0.5 H₂O: C, 62.73; H, 3.72; N, 6.15. Found: C, 62.62; H, 3.62; N, 5.68.

### Example 37

### 7-(4-Fluorophenyl)-6-(4-(methylsulphonyl)phenyl)-3-phenylpyrazolo[5,1-b][1,3]oxazole

The desired compound was prepared according to the method of Example 36A and 36B, substituting 2-bromoacetophenone in place of 4'-fluoro-2-bromoacetophenone (yield: 80 mg, 47%). MP 151-154 °C;MS (APCI+) m/z 433 (M+H)⁺; (APCI-) m/z 467 (M+Cl)⁻; ¹H NMR (300 MHz, DMSO-d₆) δ 3.27 (s, 3H), 7.30 (m, 2H), 7.41 (m, 2H), 7.52 (t, J = 9 Hz, 1H), 7.61 (t, J = 9 Hz, 2H), 7.80 (d, J = 9 Hz, 2H), 7.99 (d, J = 9 Hz, 2H), 8.24 (m, 2H), 8.85 (s, 1H); Anal. calc. for C₂₄H₁₇FN₂O₃S•H₂O: C, 63.99; H, 4.25; N, 6.21. Found: C, 64.02; H, 3.96; N, 5.41.

### Example 38

### 7-(4-Fluorophenyl)-6-(4-(methylsulphonyl) phenyl)-2,3-diphenylpyrazolo[5,1-b][1,3]oxazole

The desired compound was prepared according to the method of Example 36A and 36B, substituting desyl bromide in place of p-fluorophenacyl bromide. (yield: 80 mg, 31%). MP 228-229 °C; MS (APCI+) m/z 509 (M+H)⁺; (APCI-) 507 (M-H)⁻; ¹H NMR (300 MHz, DMSO-d₆) δ 3.24 (s, 3H), 7.30 (t, J = 9 Hz, 2H), 7.48 (m, 5H), 7.61 (m, 5H), 7.76 (d, J = 9 Hz, 2H), 7.85 (m, 2H), 7.95 (d, J = 9 Hz, 2H).

### Example 39

### 3-(2-Adamantyl)-7-(4-fluorophenyl)-6-(4-(methylsulphonyl)phenyl)pyrazolo[5,1-b][1,3]oxazole

The desired product was prepared as described in Example The desired compound was prepared according to the method of Example 36A and 36B, substituting adamantyl bromomethyl ketone in place of p-fluorophenacyl bromide. (yield: 190 mg, 77%). MP 128-131 °C; MS (APCI+) m/z 491 (M+H)⁺; (APCI-) m/z 489 (M-H)⁻, 525 (M+Cl)⁻; ¹H NMR (300 MHz, DMSO-d₆) δ 1.80 (m, 6H), 2.12 (m, 9H), 3.25 (s, 3H), 7.23 (t, J = 9 Hz, 2H), 7.34 (m, 2H), 7.74 (d, J = 9 Hz, 2H), 7.95 (m, 3H).

### Example 40

### 3-(Tert-butyl)-7-(4-fluorophenyl)-6-(4-(methylsulphonyl)phenyl)pyrazolo[5,1-b][1,3]oxazole

The desired compound was prepared according to the method of Example 36A and 36B, substituting 1-*b*romopinacolone in place of 4'-fluoro-2-bromoacetophenone (yield: 120 mg, 58%). MP 181-182 °C; MS (APCI+) m/z 413 (M+H)⁺; (APCI-) m/z 413 (M)⁺, 447 (M+Cl)⁻; ¹H NMR (300 MHz, DMSO-d₆) δ 1.47 (s, 9H), 3.24 (s, 3H), 7.23 (t, J = 9 Hz, 2H), 7.36 (m, 2H), 7.74 (d, J = 9 Hz, 2H), 7.95 (m, 3H); Anal. calc. for C₂₂H₂₁FN₂O₃S•0.5 H₂O: C, 62.69; H, 5.26; N, 6.64. Found: C, 62.87; H, 4.96; N, 6.56.

### Example 41

### 7-(4-Fluorophenyl)-2-methyl-6-(4-(methylsulphonyl) phenyl)-3-phenylpyrazolo[5,1-b][1,3]oxazole

The desired compound was prepared according to the method of Example 36A and 36B, substituting 2-bromopropiophenone in place of p-fluorophenacyl bromide. (yield: 180 mg, 80%). MP 234-236 °C; MS (APCI+) m/z 447 M+H)⁺; (APCI-) m/z 445 (M-H)⁻; ¹H NMR (300 MHz, DMSO-d₆) δ 2.66 (s, 3H), 3.25 (s, 3H), 7.27 (t, J = 9 Hz, 2H), 7.40 (m, 2H), 7.53 (m, 1H), 7.62 (t, J = 9 Hz, 2H), 7.77 (d, J = 9 Hz, 2H), 8.00 (m, 4H); Anal. calc. for C₂₅H₁₉FN₂O₃S•0.5 H₂O: C, 65.92; H, 4.42; N, 6.14. Found: C, 65.91; H, 4.61. N, 5.59.

### Example 42

### 3-(4-Fluorophenyl)-2-(4-(methylsulphonyl) phenyl)-5,6,7,8-tetrahydropyrazolo[5,1-b][1,3]benzoxazole

### 42A. 2-{[4-(4-Fluorophenyl)-3-(4-(methylsulphonyl) phenyl)-1H-pyrazol-3-yl]oxy}cyclohexanone

To a mixture of 1*H*-pyrazol-3-ol, (166 mg, 0.5 mmol), prepared according to the method of Example 5C, and K₂CO₃ (69 mg, 0.5 mmol) in DMF (20 mL) at 50 °C, was added dropwise a solution of 2-chlorocyclohexanone (0.059 mL, 0.5 mmol) in DMF (10 mL). The reaction was maintained at 50 °C for the next 2 hours. Water was added to the mixture and it was extracted with ethyl acetate. The acetate extract was washed with water, brine, dried over MgSO₄ and concentrated in vacuo. Column chromatography of the residue (silica gel, ethyl acetate) provided 2-{(4-(4-fluorophenyl)-3-(4-(methyl-sulphonyl)phenyl)-1*H*-pyrazol-3-yl]oxy}cyclohexanone (yield: 150 mg, 73%).

### 42B. 3-(4-Fluorophenyl)-2-(4-(methylsulphonyl) phenyl)-5,6,7,8-tetrahydropyrazolo[5,1-b][1,3]benzoxazole

A mixture of the pyrazole derivative (100 mg, 0.21 mmol), prepared according to the method of Example 42A, and pyridinium tosylate (15 mg, 0.06 mmol) in toluene (25 mL) and acetic acid (10 mL) was refluxed using Dean-Stark trap for 72 hours. The mixture was concentrated in vacuo and the residue chromatographed (silica gel, 1:2 hexane-ethyl acetate) to provide the desired product (yield: 70 mg, 83%). MP 183-184 °C; MS (APCI+) m/z 411 (M+H)⁺; (APCI-) m/z 445 (M+Cl)⁻; ¹H NMR (300 MHz, DMSO-d₆) δ 1.89 (m, 4H), 2.75 (m, 4H), 3.25 (s, 3H), 7.23 (m, 2H), 7.37 (m, 2H), 7.72 (m, 2H), 7.96 (m, 2H); Anal. calc. for C₂₂H₁₉FN₂O₃S: C, 64.37; H, 4.66; N, 6.82. Found: C, 64.27; H, 4.49; N, 6.51.

### Example 43

### 2,7-Bis(4-fluorophenyl)-3-methyl-6-(4-(methylsulphonyl) phenyl)pyrazolo[5,1-b][1,3]oxazole

The desired compound was prepared according to the method of Example 36 A and 36B, substituting 1-chloro-1-(4-fluorophenyl)acetone in place of p-fluorophenacyl bromide. (yield: 150 mg, 76%). MP 257-259 °C; MS (APCI+) m/z 465 (M+H)⁺; (APCI-) m/z 464 (M)⁺, 499 (M+Cl)⁻; ¹H NMR (300 MHz, DMSO-d₆) δ 2.66 (s, 3H), 3.25 (s, 3H), 7.28 (t, J = 9 Hz, 2H), 7.43 (m, 4H), 7.80 (m, 4H), 7.97 (d, J = 9 Hz, 2H); Anal. calc. for C₂₅H₁₈F₂N₂O₃S•0.25 H₂O: C, 64.02; H, 3.97; N, 5.97. Found: C, 63.76; H, 3.76; N, 5.89.

### Example 44

### 7-(4-Fluorophenyl)-3-(2-thienyl)-2-methyl-6-(4-(methylsulphonyl)phenyl)pyrazolo[5,1-b][1,3]oxazole

The desired compound was prepared according to the method of Example 36A and 36B, substituting 2-chloro-1-(2-thienyl)propan-1-one for 4'-fluoro-2-bromoacetophenone (yield: 93 mg, 68%). MP 240-241 °C; MS (APCI+) m/z 453 (M+H)⁺; (APCI-) m/z 451 (M-H)⁻; ¹H NMR (300 MHz, DMSO-d₆) δ 2.70 (s, 3H), 3.24 (s, 3H), 7.30 (m, 3H), 7.40 (m, 2H), 7.78 (d, J = 9 Hz, 2H), 7.86 (m, 1H), 7.96 (m, 3H); Anal. calc. for C₂₃H₁₇FN₂O₃S₂: C, 61.04; H, 3.78; N, 6.19. Found: C, 60.94; H, 3.85; N, 6.05.

### Example 45

### 3-(5-Chloro-2-thienyl)-7-(4-fluorophenyl)-2-methyl-6-(4-(methylsulphonyl)phenyl)pyrazolo[5,1-b][1,3]oxazole

The desired compound was prepared according to the method of Example 36A and 36B, substituting 2-chloro-1-(5-chloro-2-thienyl)propan-1-one in place of 4'-fluoro-2-bromoacetophenone (yield: 30 mg, 53%). MP 222-223 °C; MS (APCI+) m/z 487 (M+H)⁺; (APCI-) m/z 485 (M-H)⁻, 521 (M+Cl)⁻; ¹H NMR (300 MHz, DMSO-d₆) δ 2.65 (s, 3H), 3.24 (s, 3H), 7.27 (m, 2H), 7.38 (m, 3H), 7.77 (m, 3H), 7.95 (m, 2H); Anal. calc. for C₂₃ H₁₆ClFN₂O₃S₂•0.5 H₂O: C, 55.69; H, 3.45; N, 5.64. Found: C, 55.58; H, 3.31; N, 5.48.

### Example 46

### 6--(4-(Methylsulphonyl)phenyl)-7-(4-fluorophenyl)-3-methyl-2-[3-(trifluoromethyl]phenyl]pyrazolo[5,1-b][1,3]oxazole

The desired compound was prepared according to the method of Example 36A and 36B, substituting 1-chloro-1-(3-trifluomethylphenyl)acetone in place of 4'-fluoro-2-bromoacetophenone (yield: 50 mg, 19%). MP 194-195 °C; ¹H NMR (300 MHz, DMSO-d₆) δ 2.73 (s, 3H), 3.26 (s, 3H), 7.30 (t, J = 9 Hz, 2H), 7.45 (m, 2H), 7.80 (m, 4H), 7.98 (m, 3H), 8.08 (m, 1H); MS (APCI+) m/z 515 (M+H)⁺; (APCI-) m/z 514 (M)⁺, 549 (M+Cl)⁻; Anal. calc. for C₂₆H₁₈F₄N₂O₃S: C, 60.69; H, 3.52; N, 5.44. Found: C, 60.49; H, 3.49; N, 5.30.

### Example 47

### 3-(4-Chloro-3-methylphenyl)-7-(4-fluorophenyl)-6-(4-(methylsulphonyl)phenyl)pyrazolo[5,1-b][1,3]oxazole

The desired compound was prepared according to the method of Example 36A and 36B, substituting 4'-chloro-3'-methyl-2-bromoacetophenone in place of 2-bromoacetophenone (yield: 200 mg, 74%). MP 202-205 °C; ¹H NMR (300 MHz, DMSO-d₆) δ 2.4 (s, 3H), 3.27 (s, 3H), 7.30 (m, 2H), 7.41 (m, 2H), 7.62 (d, 1H), 7.8 (m, 2H), 7.99 (m, 2H), 8.15 (m, 2H), 8.9 (s, 1H); MS (DCI-NH₃) m/z 481(M+H)⁺; 498 (M+NH₄)⁺; Anal. calc. for C₂₅H₁₈ClFN₂O₃S: C, 62.43.; H, 3.77; N, 5.82. Found: C, 62.90; H, 4.05; N, 5.82.

### Example 48

### 7-(4-Fluorophenyl)-6-(4-methylsulfonylphenyl)-2,3-dimethylpyrazolo[5,1-b][1,3]oxazole

The desired compound was prepared according to the method of Example 36A and 36B, substituting 2-bromo-3-butanone in place of 4'-fluoro-2-bromoacetophenone (yield: 155 mg, 55%). MP 158-160 °C; MS (DCI-NH₃) m/z 385 (M+H)⁺; 402 (M+NH₄)⁺; ¹H NMR (300 MHz, DMSO-d₆) δ 2.4 (s, 3H), 3.25 (s, 3H) 7.25 (m, 2H), 7.35 (m, 2H), 7.75 (d, 1H), 7.9 (m, 2H); Anal. calc. for C₂₀H₁₇FN₂O₃S: C, 62.49.; H, 4.46; N, 7.29. Found: C, 62.04, H, 4.82, N, 6.76

### Example 49

### 7-(4-Fluorophenyl)-6-(4-methylsulfonylphenyl) -3-(cyclohexylmethyl)pyrazolo[5,1-b][1,3]oxazole

The desired compound was prepared according to the method of Example 36A and 36B, substituting 1-bromo-3-cyclohexylacetone in place of p-fluorophenacyl bromide. (yield: 78 mg, 34%). MP 171-173 °C; MS (DCI-NH₃) m/z 453 (M+H)⁺; ¹H NMR (300 MHz, DMSO-d₆) δ 1.05 (m, 6H), 1.65 (m, 5H), 2.7 (d, 2H),3.25 (s, 3H), 7.23 (t, J = 9 Hz, 2H), 7.34 (m, 2H), 7.74 (d, J = 9 Hz, 2H), 7.95 (m, 3H); Anal. calc. for C₂₅H₂₅FN₂O₃S•0.5 H₂O: C, 65.05.; H, 5.67; N, 6.06. Found: C, 65.31, N,5.55, N, 5.74.

### Example 50

### 7-(4-Fluorophenyl)-6-(4-(methylsulphonyl)phenyl) -3-(trifluoromethyl)pyrazolo[5,1-b][1,3]oxazole

### 50A. 1,1,1-Trifluoro-3-{[4-(4-fluorophenyl)-3-(4-(methylsulphonyl)phenyl)-1H-pyrazol-5-yl]oxy}acetone

To a mixture of 1*H*-pyrazol-3-ol (250 mg, 0.75 mmol), prepared according to the method of Example 5C, and K₂CO₃ (104 mg, 0.75 mmol) in DMF (30 mL) at 40 °C was added dropwise a solution of 3-bromo-1,1,1-trifluroacetone (0.078mL, 0.75 mmol) in DMF (10 mL). The resulting mixture was stirred at 40 °C for 3 hours. The mixture was then poured into water and extracted with ethyl acetate. The acetate layer was washed with water, brine, dried over anhydrous MgSO₄ and concentrated *in vacuo*. The residue was chromatographed (silica gel, 3:7 hexane:ethyl acetate) to provide 1,1,1-trifluoro-3-{[4-(4-fluorophenyl)-3-(4-(methylsulphonyl)phenyl)-1*H*-pyrazol-3-yl]oxy}acetone.(yield: 80 mg, 24%).

### 50B. 7-(4-Fluorophenyl)-6-(4-methylsulfonylphenyl) - 2-(trifluoromethyl)pyrazolo[5.1-b][1,3]oxazole

A mixture of the pyrazole compound (68 mg, 0.015 mmol), prepared according to the method of Example 50A, in toluene (10 mL) and polyphosphoric acid (2 drops) was refluxed for 18 hours. The mixture was concentrated *in vacuo* and the residue was dissolved in ethyl acetate. The organic layer was washed with 10% bicarbonate, brine, dried with MgSO₄ and concentrated *in vacuo*. The residue was chromatographed (silica gel, 6:4 hexane-ethyl acetate) to provide the desired product (yield: 18 mg, 28%). MP 186-189 °C; MS (APCI+) m/z 425 (M+H)⁺; (APCI-) m/z 423 (M-H)⁻, 459 (M+Cl)⁻; ¹H NMR (300 MHz, DMSO-d₆ δ δ 3.25 (s, 3H), 7.29 (m, 2H), 7.4 (m, 2H), 7.73 (d, 2H),7.95 (d, J = 9 Hz, 2H) 9.12 (s, 1H).

### Example 51

### Ethyl 7-(4-Fluorophenyl)-6-(4-methylsulfonylphenyl) -3-methylpyrazolo[5,1-b][1,3]oxazole-2-carboxylate

### 51A. Ethyl 2-{(4-(4-fluorophenyl)-5-(4-(methylsulphonyl) phenyl)-1H-pyrazol-3-yl]oxy}-3-oxobutanoate

A solution of 1*H*-pyrazol-3-ol derivative (332 mg, 1 mmol), prepared according to the method of Example 5C, and K₂CO₃ (138 mg, I mmol) in DMF (25 mL) at 0 °C was treated dropwise with a solution of ethyl 2-chloro-3-oxobutanoate (0.15 mL, 1 mmol) in DMF (10 mL), and the resulting mixture was stirred at 0 °C for 5 hours and at room temperature for 4 hours. The mixture was then treated with 10% Citric acid and extracted with ethyl acetate. The acetate layer was washed with water, brine, dried over MgSO₄, and concentrated *in vacuo* to provide 450 mg (98%) of O-alkylated product.

### 51B. Ethyl 7-(4-fluorophenyl)-6-(4-methylsulfonylphenyl) -3-methylpyrazolo[5,1-b][1,3]oxazole-2-carboxylate

The O-alkylated intermediate was dissolved in toluene (100 mL) and acetic acid (30 mL) and treated with p-toluenesulphonic acid hydrate (50 mg). The mixture was then refluxed for 10 hours, using a Dean-Stark trap to remove water. The volatile organic material was removing *in vacuo*. The residue was dissolved in ethyl acetate and washed with 10% sodium bicarbonate. Purification by chromatography (silica gel, 1:1 hexane-ethyl acetate) provided the desired product (yield: 400 mg, 90%). MP 203-204 °C; MS (APCI+) m/z 443 (M+H)⁺; (APCI-) m/z 441 (M-H)⁻, 477 (M+Cl)⁻; ¹H NMR (300 MHz, DMSO-d₆) δ 1.34 (t, J = 7 Hz, 3H), 2.73 (s, 3H), 3.26 (s, 3H), 4.38 (q, J = 7 Hz, 2H), 7.35 (m, 4H), 7.74 (d, J = 9 Hz, 2H), 7.95 (d, J = 9 Hz, 2H); Anal. calc. for C₂₂H₁₉FN₂O₅S•0.5 H₂O; C, 58.52; H, 4.46; N, 6.20. Found: C, 58.63; H, 4.21; N, 6.11.

### Example 52

### 3-(4-Fluorophenyl)-2-(4-methylsulfonylphenyl)-7,8-dihydro-5H-pyrazolo[5,1-b]pyrano[4,3-d][1,3]oxazole

### 52A. 3-(4-Fluorophenyl)-2-(4-(methylsulphonyl)phenyl) -4a,5,7,8-tetrahydro-8a-ethoxy-pyrazolo[5,1-b]pyrano[4,3-d][1,3]oxazole

A solution of 3-[(4-(4-Fluorophenyl)-5-(4-(methylsulphonyl)phenyl)-1*H*-pyrazol-3-yl)oxy]tetrahydro-4*H*-pyran-4-one, prepared according to the method of Example 23B, (340 mg, 0.8 mmol) in ethanol (120 mL) was treated with pyridinium p-toluenesulphonate (30 mg). The resulting mixture was refluxed at 75 °C for 12 hours. The mixture was then concentrated *in vacuo*, and the residue was chromatographed (silica gel, 1:2 hexanes-ethyl acetate) to provide 230 mg (63%) of cyclic aminal intermediate.

### 52B. 3-(4-Fluorophenyl)-2-(4-methylsulfonylphenyl) -7,8-dihydro-5H-pyrazolo[5,1-b]pyrano[4,3-d][1,3]oxazole

The aminal intermediate, prepared according to the method of Example 52A, was dissolved in toluene (30 mL) and acetic acid (5 mL) and treated with pyridinium p-toluenesulphonate (10 mg) at reflux for 6 hours. The mixture was concentrated *in vacuo* and purified by chromatography (silica gel, 1:2 hexanes-ethyl acetate) to provide the desired product (yield: 100 mg; 31%). MP 200-201 °C; ¹H NMR (300 MHz, DMSO-d₆) δ 2.98 (m, 2H), 3.25 (s, 3H), 4.03 (t, J = 6 Hz, 2H), 4.75 (s, 2H), 7.26 (t, J = 9 Hz, 2H), 7.37 (m, 2H), 7.74 (d, J = 9 Hz, 2H), 7.95 (d, J = 9 Hz, 2H); MS (APCI+) m/z 413 (M+H)⁺; (APCI-) m/z 447 (M+Cl)⁻; Anal. calc. for C₂₁H₁₇FN₂O₄S•0.5 H₂O: C, 59.84; H, 4.30; N, 6.64. Found: C, 60.02; H, 4.22; N, 6.53.

### Example 53

### 3-(4-Fluorophenyl)-2-(4-methylsulfonylphenyl) -7,8-dihydro-5H-pyrazolo[5,1-b]thiopyrano[4,3-d][1,3]oxazole

The desired compound was prepared according to the method of Example 52, substituting 3-bromo-tetrahydro-4*H*-thiopyran-4-one in place of 3-bromo-tetrahydro-4*H*-pyran-4-one (yield: 50 mg, 26%). MP 178-180 °C; MS (APCI+) m/z 429 (M+H)⁺; (APCI-) m/z 428 (M-H)⁻, 463 (M+Cl)⁻; ¹H NMR (300 MHz, DMSO-d₆) δ 3.06 (m, 2H), 3.50 (m, 2H), 3.90 (s, 2H), 7.25 (m, 2H), 7.34 (m, 2H), 7.74 (d, J = 9 Hz, 2H), 7.95 (d, J = 9 Hz, 2H); Anal. calc. for C₂₁H₁₇FN₂O₃S₂•0.75 H₂O: C, 57.06; H, 4.21; N, 6.33. Found: C, 57.08; H, 4.09; N, 6.14.

### Example 54

### 2-Cyano-7-(4-fluorophenyl)-3-methyl-6-(4-(methylsulphonyl)phenyl)pyrazolo[5,1-b][1,3]oxazole

### 54A. 7-(4-Fluorophenyl)-3-methyl-6-(4-(methylsulphonyl) phenyl)pyrazolo[5,1-b][1,3]oxazole-2-carboxylic acid

A solution of the ester prepared according to the method of Example 51B, (300 mg, 0.68 mmol) in dioxane (13 mL) and ethanol (7 mL) was treated with 1 N sodium hydroxide (1.4 mL, 1.4 mmol) and stirred at room temperature for 1 hour. Water (10 mL) was added, and the organics were removed *in vacuo*. The water solution was acidified with 10% citric acid, to pH 3, and the solid filtered, washed with water, and dried *in vacuo* to provide 277 mg (98%) of acid.

### 54B. 7-(4-Fluorophenyl)-3-methyl-6-(4-(methylsulphonyl)phenyl)pyrazolo[5,1-b][1,3]oxazole-2-carboxamide

A mixture of acid prepared according to the method of Example 54A, (190 mg, 0.46 mmol) and N-hydroxysuccinimide (57 mg, 0.46 mmol) in THF (15 mL) was treated dropwise with a solution of dicyclohexylcarbodimide (DCC) (100 mg, 0.46 mmol) in THF (5 mL). The mixture was stirred for 5 hours. A precipitate, dicyclohexylurea, appeared. The precipitate was removed by filtration and the filtrate treated with 10% ammonium hydroxide (15 mL), at 0 °C. The mixture was left at room temperature for 3 hours and concentrated *in vacuo*. The residue was extracted with ethyl acetate to provide 200 mg (∼100 %) of crude amide.

### 54C. 2-Cyano-7-(4-fluorophenyl)-3-methyl-6-(4-(methylsulphonyl)phenyl)pyrazolo[5,1-b][1,3]oxazole

A solution of amide prepared according to the method of Example 54B, in DMF (10 mL) at room temperature was treated with cyanuric chloride (92 mg, 0.5 mmol), in one portion. The mixture was stirred at 50 °C for 20 minutes and ethyl acetate was added. The resulting solution was washed with water, brine, dried over MgSO₄, and concentrated *in vacuo*. The residue was chromatographed (silica gel, 19:1 CH₂Cl₂-ethyl acetate) to provide the desired product (yield: 100 mg; 54%). MP 236-238 °C; MS (APCI+) m/z 396 (M+H)⁺; (APCI-) m/z 430 (M+Cl)⁻; ¹H NMR (300 MHz, DMSO-d₆) δ 2.65 (s, 3H), 3.28 (s, 3H), 7.29 (m, 2H), 7.37 (m, 2H), 7.75 (d, J = 9 Hz, 2H), 7.98 (d, J = 9 Hz, 2H); Anal. calc. for C₂₀H₁₄FN₃O₃S•0.25 H₂O: C, 60.06; H, 3.65; N, 10.50. Found: C, 60.12; H, 3.51; N, 10.49.

### Example 55

### 6-(4-(Aminosulphonyl)phenyl-3-(tert-butyl)-7-(4-fluorophenyl)pyrazolo[5,1-b][1,3]oxazole

### 55A. 4-(N',N'-dimethylaminomethyleneaminosulphonyl)benzoyl chloride

A suspension of 4-carboxybenzenesulphonamide (5.03 g, 25 mmol) in 2 M solution of oxalyl chloride in CH₂Cl₂ (30 mL, 60 mmol) at room temperature was treated dropwise with DMF (2.1 mL, 26 mmol). The resulting mixture was refluxed at 40 °C for 12 hours. The mixture was concentrated *in vacuo* to provide the crude acid chloride, which was used directly in the next step.

### 55B. Ethyl 3-(4-(N',N'-dimethylaminomethyleneaminosulphonyl) phenyl)-2-(4-fluorophenyl)-3-oxo-propionate

A solution of ethyl 4-fluorophenylacetate (4.5 g, 25 mmol) in THF (50 mL) at -78 °C was treated with 1 N LiNTMS₂ (26 mL, 26 mmol). After 20 minutes the acid chloride, prepared according to the method of Example 55A,was added in portions, and the reaction mixture was stirred for the 3 hours. The mixture was then concentrated *in vacuo,* 10% citric acid was added, and extracted with ethyl acetate. The acetate layer was washed with water, brine, dried over anhydrous MgSO₄, and concentrated *in vacuo*. Purification by column chromatography (silica gel, ethyl acetate) provided the desired keto-ester (yield: 5 g; 50%). MS (APCI+) m/z 421 (M+H)⁺; (APCI-) m/z 419 (M-H)⁻, 455 (M+Cl)⁻; ¹H NMR (300 MHz, DMSO-d₆) δ 1.15 (t, J = 7 Hz, 3H), 2.90 (s, 3H), 3.14 (s, 3H), 4.14 (q, J = 7 Hz, 2H), 6.25 (s, 1H), 7.20 (t, J = 9 Hz, 2H), 7.43 (m, 2H), 7.90 (d, J = 9 Hz, 2H), 8.14 (d, J = 9 Hz, 2H), 8.23 (s, 1H).

### 55C. 5-(4-(Aminosulphonyl)phenyl)-4-(4-fluorophenyl)-3-hydroxypyrazole

A mixture of the keto-ester (5g, 12 mmol) prepared according to the method of Example 55B, hydrazine hydrate (1.5 mL, 25 mmol), and acetic acid (1.8 mL, 30 mmol) in dioxane (120 mL) was refluxed for 4 hours. The mixture was then concentrated, and the residue dissolved in ethyl acetate and washed with water and brine. Removal of acetate *in vacuo* and purification of the residue by chromatography (silica gel, CH₂Cl₂₋ethanol 4:1) provided the desired pyrazole (yield: 3.4 g; ∼85%). MS (APCI+) m/z 334 (M+H)⁺; (APCI-) m/z 332 M-H)⁻, 368 (M+Cl)⁻; ¹H NMR (300 MHz, DMSO-d₆) δ 7.15 (t, J = 9 Hz, 2H), 7.35 (m, 2H), 7.40 (s, 2H), 7.49 (d, J = 9 Hz, 2H), 7.80 (d, J = 9 Hz, 2H).

### 55D. 5-(4-Aminosulphonylphenyl)-4-(4-fluorophenyl)-3-trimethylacetylmethoxypyrazole

A solution of the pyrazole prepared according to the method of Example 55C, (666 mg, 2 mmol) and K₂CO₃ (276 mg, 2 mmol) in DMF (25 mL) at 50 °C was treated dropwise with bromopinacolone (0.28 mL, 2 mmol) in DMF (5 mL). The mixture was stirred at 50 °C for 30 minutes and poured into 10% citric acid and extracted with ethyl acetate. The extract was concentrated *in vacuo* to provide the crude O-alkylated pyrazole (yield: 758 mg, 57%). MS (APCI+) m/z 432 (M+H)⁺; (APCI-) m/z 430 (M-H)⁻, 466 M+Cl)⁻.

### 55E. 2-(4-Aminosulphonylphenyl)-6-t-butyl-3-(4-fluorophenyl)pyrazolo[5,1-b]oxazole

A mixture of the O-alkylated pyrazole derivative prepared according to the method of Example 55D, (216 mg, 0.5 mmol), p-toluenesulfonic acid hydrate (30 mg) in acetic acid (20 mL), and toluene (80 mL) was refluxed using a Dean-Stark trap to remove water for 4 hours. The solvents were removed and the product concentrated *in vacuo*. The residue was dissolved in ethyl acetate, washed with water, brine, dried over MgSO₄, and concentrated *in vacuo*. The residue was chromatographed (silica gel, 1:1 hexanes-ethyl acetate) to provide the desired product (yield: 168 mg; 80%). MP 196-200 °C; MS (APCI+) m/z 414 (M+H)⁺; (APCI-) m/z 412 (M-H)⁻, 448 (M+Cl)⁻; ¹H NMR (300 MHz, DMSO-d₆) δ 1.50(s, 9H), 7.25 (t, J = 9 Hz, 2H), 7.33 (m, 2H), 7.43 (s, 2H), 7.65 (d, J = 9 Hz, 2H), 7.83 (d, J = 9 Hz, 2H), 7.95 (s, 1H); Anal. calc. for C₂₁H₂₀FN₃O₃S•0.25 H₂O: C, 60.34; H, 4.94; N; 10.05. Found: C, 60.47; H, 5.14; N, 9.45.

### 55F. 2-(4-N-Acetylaminosulphonylphenyl) -6-t-butyl-3-(4-fluorophenyl)pyrazolo[5,1-b]oxazole

The desired product was isolated from the reaction mixture of Example 55D by chromatography (yield: 40 mg, 17%). MP 226-230 °C; MS (APCI+) m/z 456 (M+H)⁺; (APCI-) m/z 454 (M-H)⁻; ¹H NMR (300 MHz, DMSO-d₆) δ 1.47 (s, 9H), 1.94 (s, 3H), 7.23 (t, J = 9 Hz, 2H), 7.35 (m, 2H), 7.71 (d, J = 9 Hz, 2H), 7.92 (s+d, 3H), 12.11 (br s, 1H).

### EXAMPLE 56

### 3-(Tert-butyl)-6-(4-(methylsulphonyl)phenyl)-7-phenylpyrazolo[5,1-b][1,3]oxazole

A mixture of 5-(4-(methylsulphonyl)phenyl)-4-phenyl-3-trimethylacetylmethoxy-pyrazole prepared according to the Example 32B [ (100 mg, 0.24 mmol), p-toluenesulfonic acid hydrate (25 mg) in toluene (40 mL), and acetic acid (15 mL) was refluxed using a Dean-Stark trap to remove water, for 12 hours. The reaction mixture was concentrated *in vacuo* and the residue chromatographed (silica gel, 1:1 hexane/ethyl acetate) to provide the desired product (yield: 97 mg; 99%). MP 187-180 °C; MS (APCI+) m/z 395 (M+H)⁺; (APCI-) m/z 393 (M-H)⁻, 429 (M+Cl)⁻; ¹H NMR (300 MHz, DMSO-d₆) δ 1.49 (s, 9H), 3.25 (s, 3H), 7.35 (m, 5H), 7.76 (d, J = 9 Hz, 2H), 7.95 (s+d, J = 9 Hz, 3H); Anal. calc. for C₂₂H₂₂N₂O₃S•0.5 H₂O: C, 65.48; H, 5.74; N, 6.94. Found: C, 65.70; H, 5.69; N, 6.65.

### EXAMPLE 57

### 4-[3,7-Bis(4-fluorophenyl)pyrazolo[5,1-b][1,3]oxazol-6-yl]benzenesulphonamide

The desired compound was prepared according to the method of Example 55E, substituting 4'-fluoro-2-bromoacetophenone in place of bromopinacolone (yield: 60 mg, 55%). MP 235-237 °C; MS (APCI+) m/z 452 (M+H)⁺; (APCI-) m/z 450 (M-H)⁻, 486 (M+Cl)⁻; ¹H NMR (300 MHz, DMSO-d₆) δ 7.26 (t, J = 9 Hz, 2H), 7.40 (m, 2H), 7.43 (s, 2H), 7.50 (d, J = 9 Hz, 2H), 7.72 (d, J = 9 Hz, 2H), 7.78 (d, J = 9 Hz, 2H), 8.30 (m, 2H), 8.80 (s, 1H); Anal. calc. for C₂₃H₁₅F₂N₃O₃S•0.25 H₂O: C, 60.58; H, 3.42; N, 9.21. Found: C, 60.62; H, 3.48; N, 8.86.

### EXAMPLE 58

### 1-[7-(4-Fluorophenyl)-3-methyl-6-(4-(methylsulphonyl) phenyl)pyrazolo[5,1-b][1,3]oxazol-2-yl]but-2-en-1-one

### 58A. 7-(4-Fluorophenyl)-6-(4-(Methylsulphonyl)phenyl) pyrazolo[5,1-b][1,3]oxazole-2-(N-methoxy-N-methyl)carboxamide

The N-hydroxysuccinimide ester of 5-carboxy-3-(4-fluorophenyl)-6-methyl-2-(4-(methylsulphonyl)phenyl)pyrazolo[5,1-b]oxazole, prepared according to the method of Example 54B (565 mg, 1.38 mmol) was treated with N-methoxy-N-methylamine hydrochloride (196 mg, 2 mmol), sodium bicarbonate (168 mg, 2 mmol) in water (15 mL), and ethyl acetate (20 mL). The reaction was continued for 8 hours at room temperature and then separated and concentrated *in vacuo*. The residue was purified by chromatography (silica gel, ethyl acetate) to provide the N-methoxy-N-methylamide (yield: 570 mg; 92%). MS (APCI+) m/z 458 (M+H)⁺; (APCI-) m/z 492 (M+Cl)⁻; ¹H NMR (300 MHz, DMSO-d₆) δ 2.68 (s, 3H), 3.25 (s, 3H), 3.29 (s, 7.97 (d, J = 9 Hz, 2H).

### 58B. 1-[7-(4-Fluorophenyl)-3-methyl-6-(4-(methylsulphonyl) phenyl)pyrazolo[5,1-b][1,3]oxazol-2-yl]but-2-en-1-one

A solution of the above N-methoxy-N-methylamide (275 mg, 0.6 mmol), prepared according to the method of Example 58A, in THF (50 mL) at room temperature was treated dropwise with a 1 M solution of allylmagnesium bromide (1.2 mL, 1.2 mmol), and the mixture was left at room temperature for 5 hours. The mixture was then quenched with a saturated solution of NH₄Cl and extracted with ethyl acetate. The extract was washed with water, brine, dried over MgSO₄, and concentrated *in vacuo*. The residue was chromatographed (silica gel, 1:1 hexanes-ethyl acetate) to provide the desired product (yield: 90 mg; 34%). MP 197-199 °C; MS (APCI+) m/z 439 (M+H)⁺; (APCI-) m/z 437 (M-H)⁻, 473 (M+Cl)⁻; ¹H NMR (300 MHz, DMSO-d₆) δ 2.00 (d-d, J = 1.5 and 7 Hz, 3H), 2.80 (s, 3H), 3.27 (s, 3H), 6.96 (d-d, J = 1.5 and 15 Hz, 1H), 7.22 (m, 1H), 7.30 (t, J = 9 Hz, 2H), 7.41 (m, 2H), 7.75 (d, J = 9 Hz, 2H), 7.97 (d, J = 9 Hz, 2H); Anal. calc. for C₂₃H₁₉FN₂O₄S: C, 63.00; H, 4.36; N, 6.38. Found: C, 63.06; H, 4.62; N, 6.13.

### Example 59

### 6-(4-(Aminosulphonyl)phenyl)-2-cyano-7-(4-Fluorophenyl)-3-methyl-pyrazolo[5,1-b][1,3]oxazole

The desired compound was prepared according to the method of Example 54C, substituting 5-(4-(aminosulphonyl)phenyl)-4-(4-fluorophenyl)-3-hydroxypyrazole prepared according to the method of Example 55C in place of 4-(4-fluorophenyl)-3-hydroxy-5-(4-(methylsulphonyl)phenyl)pyrazole (yield: 35 mg, 13%). MP 231-232 °C; MS (APCI+) m/z 397 (M+H)⁺; (APCI-) m/z 395 (M-H)^{-,} 431 (M+Cl)⁻; ¹H NMR (300 MHz, DMSO-d₆) δ 2.65 (s, 3H), 7.30 (m, 4H), 7.47 (s, 2H), 7.67 (d, J = 9 Hz, 2H), 7.88 (d, J = 9 Hz, 2H); Anal. calc. for C₁₉H₁₃FN₄O₃S•1.5 H₂O: C, 53.89; H, 3.80; N, 13.23. Found: C, 53.82; H, 3.93; N, 11.38.

### Example 60

### 7-(4-Fluorophenyl)-6-(4-(methylsulphonyl)phenyl)pyrazolo[5,1-b][1,3]oxazole

The desired compound was prepared according to the method of Example 36B, substituting bromomethyl dioxolane in place of 2-bromoacetophenone (yield: 25 mg, 10%). MP 67-70 °C; MS (DCI-NH₃) m/z 357 (M+H)⁺, m/z 374 (M+NH₄)⁺; ¹H NMR (300 MHz, DMSO-d₆) δ 3.25 (s, 3H) 7.25 (m, 2H), 7.37 (m, 2H), 7.73 (d, J = 9 Hz, 2H), 7.95 (d, J = 9 Hz, 2H), 8.22 (d, J = 9 Hz, 1H); 8.49 (d, J = 9 Hz, 1H).

### Example 61

### 3-Ethyl-7-(4-fluorophenyl)-6-(4-(methylsulphonyl)phenyl)pyrazolo[5,1-b][1,3]oxazole

The desired compound was prepared according to the method of Example 36B, substituting 1-bromo-2-butanone in place of 2-bromoacetophenone yield: 295 mg, 64%). MP 177-179 °C; MS (DCI-NH₃) m/z 357 (M+H)⁺, m/z 374 (M+NH₄)⁺; ¹H NMR (300 MHz, DMSO-d₆) δ 1.35 (t, J = 7 Hz, 3H), 2.85 (q, J = 7 Hz, 2H), 3.25 (s, 3H) 7.25 (m, 2H), 7.35 (m, 2H), 7.73 (d, J = 9 Hz, 2H), 7.95 (d, J = 9 Hz, 2H), 8.9 (s, 1H); Anal. calc. for C₂₀H₁₇FN₂O₃S: C, 62.49; H, 4.46; N, 7.29. Found C, 62.28; H, 4.36; N, 6.95.

### Example 62

### 6-(4-(aminosulphonyl)phenyl)-3-Ethyl-7-(4-fluorophenyl)pyrazolo[5,1-b][1,3]oxazole

A solution of 6-ethyl-3-(4-fluorophenyl) -2-(4-(methylsulphonyl)phenyl) pyrazolo-[5,1-b][1,3]oxazole prepared according to the method of Example 61 (113 mg, 0.29 mmol) and di-*t*-butylazodicarboxylate (67 mg, 0.29 mmol) in THF (10 mL) at -78 °C was treated dropwise with a 1 N solution of 1,1,1,3,3,3-hexamethyldisilazane (0.88 mL, 0.88 mmol) in THF. The reaction was stirred 45 minutes at -78 °C (or until a TLC indicated the disappearance of the starting material). The reaction mixture was treated with 1 N sodium hydroxide (1.5 mL) and stirred at room temperature for 18 hours. Sodium acetate (0.63g, 5.22 mmol) was added, followed by addition of hydroxylamine-O-sulphonic acid (630 mg, 5.22 mmol) and water (1.5 mL). The resulting mixture was stirred at room temperature for 18 hours and extracted with ethyl acetate. The extract was washed with water, brine, dried over MgSO₄, and concentrated *in vacuo*. The residue was purified by chromatography (silica gel, 95:5 CH₂Cl₂:methanol) to provide the desired product (yield: 45 mg; 41%). MP 234-236 °C; MS (DCI-NH₃) m/z 386 (M+H)⁺, m/z 403 (M+NH₄)⁺; ¹H NMR (300 MHz, DMSO-d₆) δ 1.35 (t, J = 7 Hz, 3H), 2.85 (q, J = 7 Hz, 2H), 7.25 (m, 2H), 7.35 (m, 2H), 7.43 (s, 3H), 7.65 (d, J = 9 Hz, 2H), 7.95 (d, J = 9 Hz, 2H), 8.0 (s, 1H); Anal. calc. for C₁₉H₁₆FN₃O₃S•0.25H₂O: C, 58.28; H, 4.26; N, 10.77. Found C, 58.28; H, 4.34; N, 10.17.

### Example 63

### 6-Chloro-3-(4-fluorophenyl)-2-(4-(methylsulphonyl) phenyl)-5H-pyrazolo[1,5-a][3,1]benzoxazine

The desired product was prepared according to the method of Example 6B, substituting 2-chloro-6-fluorobenzyl chloride in place of 1-bromo-2-(bromomethyl)benzene (yield: 150 mg, 55%). MP 186-187 °C; ¹H NMR (300 MHz, DMSO-d₆) δ 3.26 (s, 3H), 5.61 (s, 2H), 7.30 (m, 4H), 7.44 (d, J = 9 Hz, 1H), 7.55 (t, J = 9 Hz, 1H), 7.74 (t, J = 9 Hz, 3H), 7.95 (d, J = 9 Hz, 2H); MS (APCI+) m/z 455 (M+H)⁺; (APCI -) m/z 454 (M)⁺; Anal. calc. for C₂₃H₁₇FN₂O₃S: C, 60.72; H, 3.54; N, 6.15. Found: C, 60.72; H, 3.54; N, 6.03.

### Example 66

### 2-(4-(Aminosulphonyl)phenyl)-3-(4-fluorophenyl)-5H-pyrazolo[1,5-b][3,1]benzoxazine

The desired material was prepared according to the method of Example 6B, substituting 5-(4-aminosulphonylphenyl)-4-(4-fluorophenyl)-3-hydroxypyrazole from Example 55C in place of 4-(4-fluorophenyl)-5-(4-methylsulphonylpheny)-1*H*-pyrazol-5-ol (yield: 150 mg, 58%). MP 230-232 °C. MS (APCI+) m/z 422 (M+H)+; (APCI -) m/z 456 (M+Cl)-; ¹H NMR (300 MHz, DMSO-d₆) δ 5.51 (s, 2H), 7.30 (m, 5H), 7.44 (m, 3H), 7.52 (t, J = 9 Hz, 1H), 7.63 (d, J = 9 Hz, 2H), 7.74 (d, J = 9 Hz, 1H), 7.82 (d, J = 9 Hz, 2H); Anal. calc. for C₂₂H₁₆FN₃O₃S•0.25 H₂O: C, 62.03; H, 3.90; N, 9.86. Found: C, 62.03; H, 3.93; N, 9.77.

### Example 68

### 1:1 Mixture of 6-Chloro-3-(4-fluorophenyl)-2-(4-(aminosulphonyl)phenyl) -5H-pyrazolo[1,5-b][3,1]benzoxazine and; 6-Fluoro-3-(4-fluorophenyl) -2-(4-(aminosulphonyl)phenyl)-5H-pyrazolo[1,5-b][3,1]benzoxazine

The products were prepared according to the method of Example 66, substituting 2-chloro-6-fluorobenzyl chloride in place of 2-bromobenzyl bromide (yield: 70 mg, 26%). MP 254-256 °C; MS (APCI+) m/z 440 (M+H)⁺; m/z 456 (M+H)⁺; (APCI -) m/z 474 (M+Cl)⁻; 490 (M+Cl)⁻; ¹H NMR (300 MHz, DMSO-d₆) δ 5.61 and 5.63 (2s, 2H), 7.30 (m, 4.5H), 7.44 (m, 2.5H), 7.57 (m, 1.5H), 7.63 (d, J = 9 Hz, 2H), 7.74 (d, J = 9 Hz, 0.5H), 7.83 (d, J = 9 Hz, 2H); Anal. calc. for C₂₂H₁₅FClN₃O₃S: C,57.96; H, 3.31; N, 9.21. Found: C, 57.61; H, 3.39; N, 8.88.

### Example 72

### 5-Ethyl-3-(4-fluorophenyl)-2-(4-(methylsulphonyl)phenyl)-5H-pyrazolo[1,5-b][3,1]benzoxazine

### 72A. 1-(2-Bromophenyl)propan-1-ol

A solution of 2-bromobenzaldehyde (1.85 g, 10 mmol) in THF (30 mL) at -70 °C was treated with 3 M solution of ethylmagnesium bromide (3.5 mL, 10.5 mmol). The mixture was warmed to room temperature for 6 hours and quenched with saturated solution of NH₄Cl. The mixture was extracted with ethyl ether to provide the crude alcohol (yield: 2.2 g; ∼100%).

### 72B. 1-(Methylsulfonyloxy)-1-(2-bromophenyl)propane

The alcohol, prepared according to the method of Example 72A, in pyridine (30 mL) at 0 °C was treated dropwise with methanesulphonyl chloride (0.9 mL, 11 mmol). The resulting mixture was stirred at room temperature for 3 hours. The mixture was poured into an ice-water bath and extracted with ethyl acetate. The acetate extract was washed with 10% citric acid, brine, dried over MgSO₄, and concentrated *in vacuo* to provide crude mesylate (yield: 3 g; ∼100%).

### 72 C. 5-Ethyl-3-(4-fluorophenyl)-2-(4-(methylsulphonyl)phenyl)-5H-pyrazolo[1,5-b][3,1]benzoxazine

A solution of 4-(4-fluorophenyl)-5-(4-(methylsuphonyl)phenyl)-1*H*-pyrazol-3-ol (332 mg, 1 mmol) and K₂CO₃ (138 mg, 1 mmol) in DMF (45 mL) at 40-50 °C was treated dropwise with a solution of the mesylate prepared according to the method of Example 72B, (300 mg, 1 mmol) in DMF (5 mL) and stirred at for 30 minutes (until starting material disappeared). Potassium carbonate (K₂CO₃, 138 mg, 1 mmol) was added, followed by the addition of CuI (25 mg). The resulting mixture was stirred at 150 °C for 8 hours, poured into water and extracted with ethyl acetate. The extract was washed with water, brine, dried over MgSO₄, and concentrated *in vacuo*. The residue was purified by chromatography to provide the desired product as a foam (yield: 110 mg; 25%). ¹H NMR (300 MHz, DMSO-d₆) δ 0.96 (t, J = 7 Hz, 3H), 1.95 (m, 2H), 3.25(s, 3H), 5.59 (t, J = 7 Hz, 1H), 7.30 (m, 5H), 7.42 (m, 1H), 7.53 (t, J = 9 Hz, 1H), 7.72 (d, J = 9 Hz, 2H), 7.78 (d, J = 9 Hz, 1H), 7.93 (d, J = 9 Hz, 2H); MS (APCI+) m/z 449 (M+H)⁺. Anal. calc. for C₂₅H₂₁FN₂O₃S: C, 66.94; H, 4.71; N, 6.24. Found: C, 66.64; H, 4.87; N, 6.06.

### EXAMPLE 73

### 3,6-Bis(4-fluorophenyl)-7-(4-methylsulfonylphenyl)pyrazolo[5,1-b][1,3]oxazole

### 73A. Ethyl 2-(4-(methylthio)phenyl)acetate

A mixture of 2-(4-(methylthio)phenyl)acetic acid (10.8 g, 60 mmol) and concentrated sulfuric acid (1 mL) in ethanol (150 mL) was refluxed for 8 hours. The reaction mixture was concentrated *in vacuo*, and the residue dissolved in ethyl ether. The ether solution was washed with 10% sodium bicarbonate, brine, dried over anhydrous MgSO₄, and concentrated *in vacuo* to provide the ethyl ester (yield: 12.1 g; 96%).

### 73B. Ethyl 3-(4-fluorophenyl)-2-(4-(methylthiophenyl)-3-oxopropanoate

1 N Lithium bis(trimethylsilyl)amide (25 mL, 25 mmol) was added dropwise to a solution of ethyl 2-(4-(methylthio)phenyl)acetate (5.05 g, 24 mmol), prepared according to the method of Example 73A, in THF (20 mL) maintained at -78 °C. After 15 minutes the mixture was treated dropwise with a solution of 4-fluorobenzoyl chloride (2.85 mL, 24 mmol) in THF (50 mL), and the resulting mixture was stirred at -78 °C for 90 minutes. The mixture was quenched with saturated NH₄Cl and extracted with ethyl acetate. The acetate layer was washed with water, brine, dried over anhydrous MgSO₄, and concentrated *in vacuo*. The residue was triturated with hexanes:ether, 15:1, to provide the desired product (yield: 7.25 g; 90%). MS (DCI-NH₃) m/z 333 (M+H)⁺, 350 (M+NH₄)⁺; ¹H NMR (300 MHz, DMSO) δ 1.18 (m, 3H), 2.45 (s, 3H), 4.15 (m, 2H), 6.15 (s, 1H), 7.23 (m, 2H), 7.35 (m, 4H), 7.85 (d, J = 9 Hz, 2H).

### 73C. 5-(4-Fluorophenyl)-4-(4-(methylthio)phenyl)-1H-pyrazol-3-ol

A mixture of ethyl 2-(4-methylthio)phenyl-2-(4-fluorobenzoyl)acetate (5.2 g, 15.6 mmol), hydrazine hydrate (0.59 mL, 18.79 mmol), and acetic acid (1.07 mL, 18.8 mmol) in dioxane (75 mL) and water (5 mL) was refluxed for 24 hours and then concentrated in *vacuo*. Water was added, and the solid was filtered and dried *in vacuo* to provide the crude product (yield: 4.7 g; 90%). MS (DCI-NH₃) m/z 301 (M+H)⁺, 318 (M+NH₄)⁺; ¹H NMR (300 MHz, DMSO-d₆) δ 2.45 (s, 3H), 7.26 (m, 6H); 7.35 (m, 2H).

### 73D. 1-(4-Fluorophenyl)-2-{[5-(4-fluorophenyl) -4-(4-(methylthio)phenyl)-1H-pyrazol-3-yl]oxy}ethan-1-one

A mixture of the hydroxypyrazole (400 mg, 1.3 mmol), prepared according to the method of Example 73C, and K₂CO₃ (184 mg, 1.3 mmol) in DMF (25 mL) at 50 °C was treated dropwise with a solution of 4'-fluoro-2-bromoacetophenone (290 mg, 1.3 mmol) in DMF (10 mL) and stirred at 50 °C for 8 hours. The mixture was poured into water and extracted with ethyl acetate. The acetate extracts were washed with water, brine, dried over anhydrous MgSO₄ and concentrated *in vacuo* to provide the crude O-alkylated derivative (yield: 280 mg; 99%).

### 73E. 3,6-Bis(4-fluorophenyl)-7-(4-(methylthio)phenyl)pyrazolo[5,1-b][1,3]oxazole

A mixture of the alkylated product prepared according to the method of Example 73D, (568 mg, 1.3 mmol), toluenesulfonic acid hydrate (285 mg, 1.5 mmol) in toluene (40 mL), and acetic acid (20 mL) was refluxed for 4 hours using a Dean-Stark trap to remove water. The mixture was concentrated *in vacuo*, and the residue was dissolved in ethyl acetate. The acetate solution was washed with 10% sodium bicarbonate, brine, dried over MgSO₄, and concentrated *in vacuo*. The residue was chromatographed (silica gel, 1:1 hexanes-ethyl acetate) to provide the desired product (yield: 225 mg; 55%). MS (DCI-NH₃) m/z 419 (M+H)⁺; ¹H NMR (300 MHz, DMSO-d₆) δ 2.5 (s, 3H), 7.26 (t, J = 9 Hz, 6H), 7.45 (m, 2H), 7.5 (m, 2H), 8.3 (m, 2H), 8.8 (s, 1H).

### 73F. 3,6-Bis(4-fluorophenyl)-7-(4-methylsulfonylphenyl)pyrazolo[5,1-b][1,3]oxazole.

A solution of methylthio the derivative prepared according to the method of Example 73E, (200 mg, 0.48 mmol) in CH₂Cl₂ (20 mL) at 0 °C was treated with 32% peracetic acid (0.22 mL) and stirred for 4 hours. The mixture was then washed with water, saturated sodium bicarbonate, brine, dried over anhydrous MgSO₄, and concentrated *in vacuo*. The residue was purified by chromatography (silica gel, ethyl acetate) to provide the desired product (yield: 135 mg; 64%). MP 289-291 °C; MS (DCI-NH₃) m/z 451 (M+H)⁺, m/z 468 (M+NH₄)⁺; ¹H NMR (300 MHz, DMSO-d₆) δ 3.25 (s, 3H), 7.36 (t, J = 9 Hz, 2H), 7.45 (t, J = 9 Hz, 2H), 7.6 (m, 4H), 7.9 (d, J = 9 Hz, 2H), 8.3 (t, J = 9 Hz, 2H), 8.85 (s, 1H); Anal. calc. for C₂₄H₁₆F₂N₂O₃S•0.5 H₂O: C, 62.73; H, 3.72; N, 6.09. Found: C, 62.55; H, 3.60; N, 5.72.

### EXAMPLE 74

### 2,6-Bis(4-fluorophenyl)-3-methyl-7-(4-(methylsulphonyl)phenyl)pyrazolo[5,1-b][1,3]oxazole

The desired product was prepared according to the method of Example 73E substituting 4'-fluoro-2-bromoacetophenone in place of 3-chloro-3-(4-fluorophenyl)-2-propanone. The resulting thio-ether product was then oxidized as described in Example 73F (yield: 120 mg, 68%). MP 289-291 °C; ¹H NMR (300 MHz, DMSO-d₆) δ 2.65 (s, 3H), 3.22 (s, 3H), 7.36 (t, J = 9 Hz, 2H), 7.45 (t, J = 9 Hz, 2H), 7.6 (m, 4H), 7.9 (d, J = 9 Hz, 2H),7.8 (m, 2H), 8.85 (s, 1H); MS (DCI-NH₃) m/z 465 (M+H)⁺, m/z 482 (M+NH₄)⁺; Anal. calc. for C₂₅H₁₈F₂N₂O₃S•0.5 H₂O: C, 63.41; H, 4.04; N, 5.91. Found: C, 63.53; H, 4.04; N, 5.91.

### Example 76

### 6-(4-Fluorophenyl)-7-(4-(methylsulphonyl)phenyl)-3-ethylpyrazolo[5,1-b][1,3]oxazole

The desired product was prepared according to the method of Example 73D-73E substituting 1-bromo-2-butanone in place of 4'-fluoro-2-bromoacetophenone. The resulting thio-ether product was then oxidized as described in Example 73F (yield: 68 mg, 10%). MP 205-208 °C; MS (DCI-NH₃) m/z 357(M+H)⁺, m/z 374 (M+NH₄)⁺; ¹H NMR (300 MHz, DMSO-d₆) δ 1.35 (t, J = 7 Hz, 3H), 2.85 (q, J = 7 Hz, 2H), 3.25 (s, 3H) 7.3 (m, 2H), 7.5 (m, 4H), 7.8 (d, J = 9 Hz, 2H), 8.04 (s, 1H); Anal. calc. for C₂₀H₁₇FN₂O₃S: C, 62.49; H, 4.46; N, 7.29. Found C, 62.28; H, 4.36; N, 6.95

### Prostaglandin Inhibition Determination

### Compound Preparation and Administration

For oral administration, test compounds were suspended on the day of use in 100% polyethyleneglycol (PEG 400) with a motorized homogenizer equipped with a Teflon-coated pestle (TRI-R Instrument, Jamaica, NY).

To compare the mean responses of the treatment groups, analysis of variance was applied. Percent inhibition values were determined by comparing the individual treatment mean values to the mean of the control group. Linear regression was used to estimate IC₅₀'s/ED₅₀'s in appropriate assays.

### EIA Determination of Prostaglandins

EIA reagents for prostaglandin determination were purchased from Perseptive Diagnostics, (Cambridge, MA). Prostaglandin E₂ (PGE₂) levels in lavage fluids were determined after the samples were dried under nitrogen and reconstituted with assay buffer. PGE₂ levels in enzyme assays or cell culture media were measured against standards prepared in the same milieu. The immunoassays were conducted as recommended by the manufacturer. The EIA was conducted in 96 well microtiter plates (Nunc Roskilde, Denmark) and optical density was measured using a microplate reader (Vmax, Molecular Devices Corp., Menlo Park, CA).

### Recombinant Human PGHS-1 and PGHS-2 Enzyme Assays

Inhibition of prostaglandin biosynthesis *in vitro* was evaluated using recombinant human Cox-1 (r-hu Cox-1) and Cox-2 (r-hu Cox-2) enzyme assays. Representative compounds dissolved in DMSO (3.3% v/v) were preincubated with microsomes from recombinant human PGHS-1 or PGHS-2 expressed in the baculovirus/Sf9 cell system (Gierse, J. K., Hauser,S. D., Creely, D. P., Koboldt, C., Rangwala, S., H., Isakson, P. C., and Seibert, K. Expression and selective inhibition of the constituitive and inducible forms of cyclooxygenase , *Biochem J*. 1995, 305: 479.), together with the cofactors phenol (2 mM) and hematin (1 µM) for 60 minutes prior to the addition of 10 µM arachidonic acid. The reaction was allowed to run for 2.5 minutes at room temperature prior to quenching with HCl and neutralization with NaOH. PGE₂ production in the presence and absence of the drug was determined by EIA analysis. The EIA was conducted in 96 well microtiter plates (Nunc Roskilde, Denmark) and optical density was measured using a microplate reader (Vmax, Molecular Devices Corp., Menlo Park, CA). EIA reagents for prostaglandin determination were purchased from Perseptive Diagnostics (Cambridge, MA). PGE₂ levels were measured against standards prepared in the same milieu. The immunoassays were conducted as recommended by the manufacturer. The data illustrating the inhibition of prostaglandin biosynthesis *in vitro* by compounds of this invention is shown in Table 1. The compounds are designated by the Example Number. Column 2 shows Cox-1 percent inhibition at the particular micromolar dose level and Column 3 shows Cox-2 percent inhibition at the particular nanomolar dose level. Values for Cox-2 inhibition that are parenthetical indicate IC₅₀ values.

**TABLE 1**

| **Example No.** | **r-hu COX1 (µM)** | **r-hu COX2 (nM)** |
|---|---|---|
| 1 | 4@100 | (720) |
| 2 | 9 @ 100 | 57 @ 10000 |
| 3 | 20 @ 100 | (90) |
| 4 | 12 @ 100 | 62 @ 10000 |
| 6 | 25 @ 100 | 82 @ 100 |
| 42 | 23 @ 100 | 86 @ 100 |
| 55 | 99 @ 10 | 88 @ 100 |
| 59 | 96 @ 100 | 51 @ 100 |
| 61 | 23 @ 100 | 94 @ 100 |
| 62 | 91 @ 100 | (2) |

### IL-1β Induced PGE₂ Production in WISH Cells

Human amnionic WISH cells were grown to 80% confluence in 48 well plates. Following removal of the growth medium and two washings with Gey's Balanced Salt Solution, 5 ng IL-1β/ml (UBI, Lake Placid, NY) was added to the cells with or without test compound in DMSO (0.01% v/v) in Neuman-Tytell Serumless Medium (GIBCO, Grand Island, NY). Following an 18 hour incubation to allow for the maximal induction of PGHS-2, the conditioned medium was removed and assayed for PGE₂ content by EIA analysis as described above.

The data illustrating the inhibition of prostaglandin biosynthesis *in vitro* by compounds of this invention is shown in Table 2. WISH cell values indicate percent inhibition at the particular nanomolar dose level.

**TABLE 2**

| **Example no.** | **WISH (nM)** |
|---|---|
| 1 | 19% at 1000 |
| 3 | 43% at 10 |
| 5 | 33% at 1000 |
| 39 | 95@1 38@0.1 |
| 41 | 52@1 33@0.1 |
| 42 | 81@0.1 37@0.01 |
| 54 | (0.20) |
| 58 | 84 @ 0.1 |
| 62 | (0.21) |

### Rat Carrageenan Pleural Inflammation (CIP) Model

Pleural inflammation was induced in male adrenalectomized Sprague-Dawley rats following the method of Vinegar *et al*., *Fed*. *Proc.* **1976,** *35*, 2447-2456. Animals were orally dosed with experimental compounds, 30 minutes prior to the intrapleural injection of 2% lambda carrageenan (Sigma Chemical Co., St. Louis MO). Four hours later the animals were euthanized and the pleural cavities lavaged with ice cold saline. The lavage fluid was then added to two volumes of ice cold methanol (final methanol concentration 66%) to lyse cells and precipitate protein. Eicosanoids were determined by EIA as described above.
The data illustrating the inhibition of prostaglandin biosynthesis *in vivo* by the compounds of this invention is shown in Table 3. Values reported are percent inhibition at 10 milligrams per kilogram body weight.

**TABLE 3**

| **Example No.** | **CIP Inh @10mpk** |
|---|---|
| 3 | 3% |
| 37 | 0% |

### Pharmaceutical Compositions

The present invention also provides pharmaceutical compositions which comprise compounds of the present invention formulated together with one or more non-toxic pharmaceutically acceptable carriers. The pharmaceutical compositions of the present invention comprise a therapeutically effective amount of a compound of the present invention formulated together with one or more pharmaceutically acceptable carriers. As used herein, the term "pharmaceutically acceptable carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as com starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the procedures and judgements well known to one skilled in the art. The pharmaceutical compositions of this invention can be administered to humans and other animals orally, rectally, parenterally, intracisternally, intravaginally, intraperitoneally, topically (as by powders, ointments, or drops), bucally, or as an oral or nasal spray.

The compounds of the present invention may be potentially useful in the treatment of several illness or disease states such as inflammatory diseases, dysmennorhea, asthma, premature labor, osteoporosis, and ankylosing spondolitis. Current Drugs Ltd, ID Patent Fast Alert, AG16, May 9, 1997.

The compounds of the present invention may also be potentially useful in the treatment of cancers, and in particular, colon cancer. Proc. Natl. Acad. Sci., 94, pp. 3336-3340, 1997.

The compounds of the present invention may be useful by providing a pharmaceutical composition for inhibiting prostaglandin biosynthesis comprising a therapeutically effective amount of a compound of formula I or a pharmaceutically acceptable salt, ester, or prodrug thereof, and a pharmaceutrically acceptable carrier.

In addition, the compounds of the present invention may be useful by providing a method for inhibiting prostaglandin biosynthesis comprising administering to a mammal in need of such treatment a therapeutically effective amount of a compound of formula I or a pharmaceutically acceptable salt, ester, or prodrug thereof.

In addition, the compounds of the present invention may be useful by providing a method for treating pain, fever, inflamation, rheumatoid arthritis, osteoarthritis, and cancer comprising administering to a mammal in need of such treatment a therapeutically effective amount of a compound of formula I.

Liquid dosage forms for oral administration include pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (such as, for example, cottonseed, groundnut, corn, germ, olive, castor, sesame oils, and the like), glycerol, tetrahydrofurfuryl alcohol, poly-ethyl-ene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, such as, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, such as, for example, a solution in 1,3-butanediol. Among the acceptable vehicles and solvents that may be employed are water, Ringer's solution, isotonic sodium chloride solution, and the like. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectable preparations.

The injectable formulations can be sterilized by any method known in the art, such as, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsulated matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides) Depot injectable formulations are also prepared by entrapping the drug in liposomes or microemulsions which are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and thus melt in the rectum or vaginal cavity and release the active compound.

Solid dosage forms for oral administration include capsules, tablets, pills, powders, and granules. In such solid dosage forms, the active compound is usually mixed with at least one inert, pharmaceutically acceptable excipient or carrier such as, for example, sodium citrate or dicalcium phosphate and/or a) fillers or extenders such as, for example, starches, lactose, sucrose, glucose, mannitol, and silicic acid, b) binders such as, for example, carboxymethylcellulose, alginates, gelatin, polyvinylpyrrolidinone, sucrose, and acacia, c) humectants such as, for example, glycerol, d) disintegrating agents such as, for example, agar-agar, calcium carbonate, potato or tapioca starch, alginic acid, certain silicates, and sodium carbonate, e) solution retarding agents such as, for example, paraffin, f) absorption accelerators such as, for example, quaternary ammonium compounds, g) wetting agents such as, for example, cetyl alcohol and glycerol monostearate, h) absorbents such as, for example, kaolin and bentonite clay, and) lubricants such as, for example, talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients such as, for example, lactose or milk sugar as well as high molecular weight polyethylene glycols and the like.

Solid compositions of a similar type may also be employed as fillers in soft and hard-filled gelatin capsules using excipients such as, for example, lactose or milk sugar as well as high molecular weight polethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulation art. In such solid dosage forms the active compound may be admixed with at least one inert diluent such as, for example, sucrose, lactose or starch. Such dosage forms may also comprise, as is normal practice, additional substances other than inert diluents, e.g., tableting lubricants and other tableting aids such as, for example, magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, eye ointments, powders and solutions are also contemplated as being within the scope of this invention.

The ointments, pastes, creams and gels may contain, in addition to an active compound of this invention, excipients such as, for example, animal and vegetable fats, oils, waxes, paraffins, starch, tragacanth, cellulose derivatives, polyethylene glycols, silicones, bentonites. silicic acid, talc and zinc oxide, or mixtures thereof.

Powders and sprays can contain, in addition to the compounds of this invention, excipients such as, for example, lactose, talc, silicic acid, aluminum hydroxide, calcium silicates and polyamide powder, or mixtures of these substances. Sprays can additionally contain customary propellants such as chlorofluorohydrocarbons.

Transdermal patches have the added advantage of providing controlled delivery of a compound to the body. Such dosage forms can be made by dissolving or dispensing the compound in a suitable medium. Absorption enhancers can also be used to increase the flux of the compound across the skin. The rate can be controlled by either providing a rate controlling membrane or by dispersing the compound in a polymer matrix or gel.

According to the methods of treatment of the present invention, a patient, such as a human or mammal, is treated by administering to the patient a therapeutically effective amount of a compound of the invention, in such amounts and for such time as is necessary to achieve the desired result. By a "therapeutically effective amount" of a compound of the invention is meant a sufficient amount of the compound to provide the relief desired, at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts. The total daily dose of the compounds of this invention administered to a human or other mammal in single or in divided doses can vary from individual to individual. An example of dosage amount may be from 0.00001 to about 1000 mg/kg body weight daily or more preferably from about 0.1 to about 100 mg/kg body weight for oral administration or 0.01 to about 10 mg/kg for parenteral administration daily. Single dose compositions may contain such amounts or submultiples thereof to make up the daily dose.

The amount of active ingredient that may be combined with the carrier materials to produce a single dosage form will vary depending upon the host treated and the particular mode of administration.

The reagents required for the synthesis of the compounds of the invention are readily available from a number of commercial sources such as Aldrich Chemical Co. (Milwaukee, WI, USA); Sigma Chemical Co. (St. Louis, MO, USA); and Fluka Chemical Corp. (Ronkonkoma, NY, USA); Alfa Aesar (Ward Hill, MA 01835-9953); Eastman Chemical Company (Rochester, New York 14652-3512); Lancaster Synthesis Inc. (Windham, NH 03087-9977); Spectrum Chemical Manufacturing Corp. (Janssen Chemical) (New Brunswick, NJ 08901); Pfaltz and Bauer (Waterbury, CT. 06708). Compounds which are not commercially available can be prepared by employing known methods from the chemical literature.

## Claims

1. A compound having the formula I below 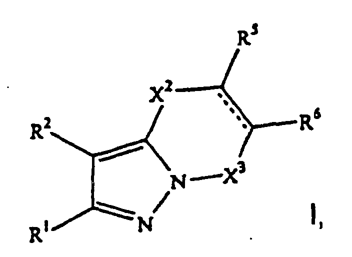 wherein one of R¹ and R² is selected from the group consisting of: 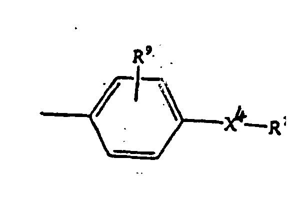 or 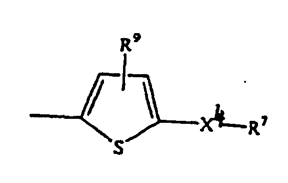 wherein R⁷ is selected from the group consisting of alkyl, amino, alkylamino, dialkylamino;
X⁴ is selected from the group consisting of -SO₂-, -SO(NR⁸)-;
R⁸ is selected from the group consisting of hydrogen, alkyl and cycloalkyl;
R⁹ is selected from the group consisting of hydrogen and halogen; and
the other of R¹ and R² is selected from the group consisting of hydroxyalkyl, halogen, alkyl, alkenyl, alkynyl, alkoxyalkyl, (NHR¹⁰)-C(O)-alkyl-, dimethylaminocarbonylalkyl, haloalkyl, cycloalkyl, cycloalkylalkyl, cycloalkenyl, cycloalkenylalkyl, aminocarbonylalkyl, aryl, heterocyclic, heterocyclic(alkyl), cyano, nitro, -Y-R¹⁰, - C(R¹¹)(R¹²)-halogen and -C(R¹¹)(R¹²)-OH ;
Y is selected from the group consisting of -O-, -S-, - C(R¹¹)(R¹²)-, -C(O)NR¹⁴-, -C(O)-, -C(O)O-, -NH-, -NR¹⁴ C(O)-, and - N(R¹³)-;
R¹⁰ is selected from the group consisting of hydrogen, alkyl, alkenyl, alkynyl, cycloalkyl, cycloalkenyl, amino, cyano, aryl, arylalkyl, heterocyclic, and heterocyclic(alkyl);
R¹¹, R¹² and R¹³ are independently selected from the group consisting of alkyl, alkenyl, cycloalkyl, cycloalkenyl, alkoxy, aryl, arylalkyl, heterocyclic, heterocyclic(alkyl) and cyano;
R¹⁴ is selected from the group consisting of hydrogen, alkyl, alkenyl, cycloalkyl, cycloalkenyl, alkoxy, aryl, arylalkyl, heterocyclic, heterocyclic(alkyl) and cyano;
X² is selected from the group consisting of -O-(CH₂)ₙ- and -S-(CH₂)ₙ-, wherein n is 0, 1 or 2;
X³ is absent, or is selected from the group consisting of -CH₂-and -C(R¹⁵)(R¹⁶)- wherein R¹⁵ and R¹⁶ are independently selected from the group consisting of hydrogen and alkyl;
R⁵ and R⁶ are selected from the group consisting of hydrogen, alkyl, substituted alkyl, alkenyl, alkynyl, aryl, cycloalkyl, heterocyclic, aryl(substituted alkyl) and arylalkyl, or R⁵ and R⁶ are taken together with the atoms to which they are attached to form a 5- to 7-membered ring, optionally aromatic, and optionally containing one or two heteroatoms selected from O, N and S, and optionally substituted with 1 to 2 groups selected from alkyl, hydroxy, halogen, oxo, haloalkyl, cyano and nitro;
provided that when n is 2, then X³ is absent and R⁵ and R⁶ taken together with the atom to which they are attached form a benzene ring;
the dashed bond represents an optional double bond;
aryl is a mono- or bicyclic carbocyclic ring system having one or two aromatic rings, wherein aryl by itself or as part of another group may be optionally substituted with one, two or three substituents selected from the group consisting of C₁-C₆ alkyl, haloalkyl, alkoxy, thioalkoxy, amino, alkylamino, dialkylamino, hydroxy, halo, mercapto, nitro, carboxaldehyde, carboxy, alkoxycarbonyl and carboxamide, or aryl may be tetrafluorophenyl or pentafluorophenyl;
heterocyclic is a 3- or 4-membered ring containing a heteroatom selected from oxygen, nitrogen and sulfur, or a 5-, 6- or 7-membered ring containing one, two or three nitrogen atoms, one nitrogen and one sulfur atom, or one nitrogen and one oxygen atom, wherein the 5-membered ring has 0-2 double bonds and the 6- and 7-membered rings have 0-3 double bonds and wherein the nitrogen atoms may be optionally quaternized, or a bicyclic group in which any of the above heterocyclic rings is fused to a benzene ring or cyclohexane ring or another heterocyclic ring as defined above, wherein heterocyclic by itself or as part of another group may be optionally substituted with one or two substituents independently selected from hydroxy, halo, oxo, C₁-C₆ alkylimino, amino, alkylamino, dialkylamino, alkoxy, alkoxyalkoxy, haloalkyl, cycloalkyl, aryl, arylalkyl, -COOH, -SO₃H and C₁-C₆ alkyl, or nitrogen-containing heterocyclics can be N-protected;
cycloalkyl is an aliphatic ring system having 3 to 10 carbon atoms and 1 to 3 rings, wherein cycloalkyl by itself or as part of another group may be optionally substituted with one, two or three substituents independently selected from C₁-C₆ alkyl, haloalkyl, alkoxy, thioalkoxy; amino, alkylamino, dialkylamino, hydroxy, halo, mercapto, nitro, carboxaldehyde, carboxy, alkoxycarbonyl and carboxamide;
cycloalkenyl is a cyclic hydrocarbon containing from 3 to 8 carbon atoms and at least one carbon-carbon double bond, wherein cycloalkenyl may be optionally substituted with one to five substituents independently selected from alkenyl, alkoxy, alkoxycarbonyl, alkoxycarbonylalkyl, alkyl, alkylcarbonyloxy, alkylcarbonyloxyalkyl, alkynyl, -C(O)-NHR¹⁰, dimethylaminocarbonyl, - alkyl-C(O)-NHR¹⁰, dimethylaminocarbonylalkyl, arylalkoxycarbonyl, arylalkoxycarbonylalkyl, arylcarbonyloxy, arylcarbonyloxyalkyl, aryloxycarbonyl, aryloxycarbonylalkyl, halo, haloalkoxy, haloalkyl, hydroxy, hydroxyalkyl, sulfamylalkyl, -NHR¹⁰ and (NHR¹⁰)alkyl;
substituted alkyl is a C₁-C₆ alkyl group substituted with 2, 3 or 4 substituents independently selected from the group consisting of alkoxy, alkoxycarbonyl, alkylcarbonyl, alkylcarbonyloxy, -C(O)-NHR¹⁰, dimethylaminocarbonyl, aryl, arylalkoxycarbonyl, arylcarbonyloxy, aryloxycarbonyl, -CF₃, cyano, cycloalkyl, halo, haloalkoxy, heterocycle, hydroxy, sulfamyl, alkylsulfonyl, arylsulfonyl and -NHR¹⁰;
or a pharmaceutically acceptable salt, ester, or prodrug thereof.

2. A compound of Claim 1 wherein R¹ is selected from the group consisting of 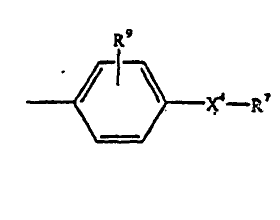 or 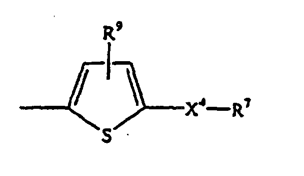 wherein R⁷, X⁴ and R⁹ are as defined in claim 1.

3. A compound of Claim 2 wherein X² is oxygen, X³ is absent or -CH₂-, and R⁵ and R⁶ form a 5- to 7-membered aromatic or non-aromatic carbocyclic ring, said carbocyclic ring optionally being mono-, di- or trisubstituted with halogen.

4. A compound of Claim 2 wherein X² is oxygen, X³ is absent or -CH₂-, R⁵ and R⁶ are independently selected from the group consisting of hydrogen, alkyl, cyano and aryl.

5. A compound of Claim 1 wherein said compound is selected from the group consisting of:
3-(4-Fluorophenyl)-2-(4-(methylsulphonyl)phenyl)-5*H*-pyrazolo[1,5-a][3,1]benzoxazine;
3-(4-Fluorophenyl)-2-(4-(methylsulphonyl)phenyl)-5,6,7,8-tetrahydropyrazolo[5,1-*b*][1,3]benzoxazole;
3-(*Tert*-butyl)-7-(4-fluorophenyl)-6-(4-(aminosulphonyl)phenyl)pyrazolo[5,1-*b*][1,3]oxazole;
7-(4-Fluorophenyl)-3-methyl-6-(4-(aminosulphonyl)phenyl)pyrazolo[5,1-*b*][1,3]oxazole-2-carbonitrile;
3-Ethyl-7-(4-fluorophenyl)-6-(4-(methylsulphonyl)phenyl)pyrazolo[5,1-*b*][1,3]oxazole;
3-Ethyl-7-(4-fluorophenyl)-6-(4-(aminosulphonyl)phenyl)pyrazolo[5,1-*b*][1,3]oxazole;
6-Chloro-3-(4-fluorophenyl)-2-(4-(methylsulphonyl)phenyl)-5*H*-pyrazolo[1,5-*a*][3,1]benzoxazine;
3-(4-Fluorophenyl)-2-(4-(aminosulphonyl)phenyl)-5H-pyrazolo[1,5-*a*][3,1]benzoxazine; and
2,6-Bis(4-fluorophenyl)-3-methyl-7-(4-(methylsulphonyl)phenyl)pyrazolo[5,1-*b*][1,3]oxazole;
or a pharmaceutically acceptable salt, ester, amide or prodrug thereof.

6. A pharmaceutical composition comprising a therapeutically effective amount of a compound of Claim 1 or a pharmaceutically acceptable salt, ester, or prodrug thereof, and a pharmaceutically acceptable carrier.

7. A compound of Claim 1 or a pharmaceutically acceptable salt, ester or prodrug thereof for use as a therapeutic agent.

8. Use of a compound of Claim 1 for manufacturing a medicament for treating pain, fever, inflammation, rheumatoid arthritis, osteoarthritis, and cancer by administration to a mammal in need of such treatment of a therapeutically effective amount.

## Patentansprüche

1. Eine Verbindung mit der Formel I unten 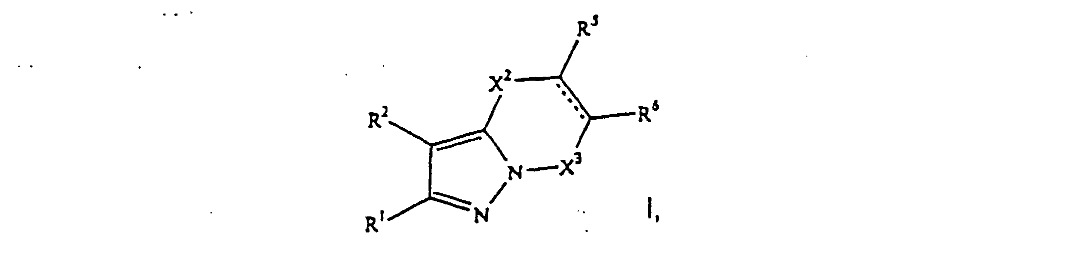 worin eins von R¹ und R² gewählt ist aus der Gruppe bestehend aus: 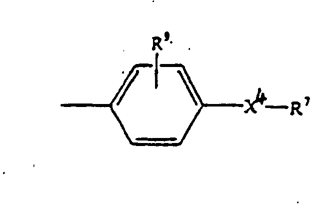 oder 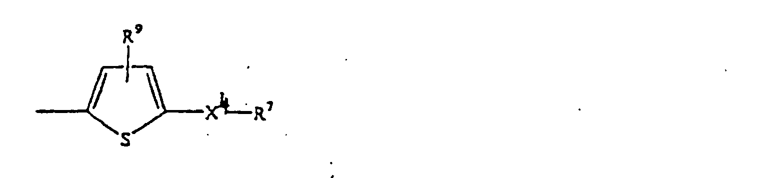 worin R⁷ gewählt ist aus der Gruppe bestehend aus Alkyl, Amino, Alkylamino, Dialkylamino;
X⁴ ist gewählt aus der Gruppe bestehend aus -SO₂-, -SO(NR⁸)-;
R⁸ ist gewählt aus der Gruppe bestehend aus Wasserstoff, Alkyl und Cycloalkyl;
R⁹ ist gewählt aus der Gruppe bestehend aus Wasserstoff und Halogen; und
das andere von R¹ und R² ist gewählt aus der Gruppe bestehend aus Hydroxyalkyl, Halogen, Alkyl, Alkenyl, Alkinyl, Alkoxyalkyl, (NHR¹⁰)-C (O)-Alkyl-, Dimethylaminocarbonylalkyl, Haloalkyl, Cycloalkyl, Cycloalkylalkyl, Cycloalkenyl, Cycloalkenylalkyl, Aminocarbonylalkyl, Aryl, Heterozyklus, Heterozyklus (alkyl) , Cyano, Nitro, -Y-R¹⁰, -C(R¹¹)(R¹²)-Halogen und -C(R¹¹)(R¹²)-OH;
Y ist gewählt aus der Gruppe bestehend aus -O-, -S-, - C(R¹¹)(R¹²)-, -C(O)NR¹⁴-, -C(O)-, -C(O)O-, -NH-, -NR¹⁴C(O)- und - N(R¹³)-;
R¹⁰ ist gewählt aus der Gruppe bestehend aus Wasserstoff, Alkyl, Alkenyl, Alkinyl, Cycloalkyl, Cycloalkenyl, Amino, Cyano, Aryl, Arylalkyl, Heterozyklus und Heterozyklus(alkyl);
R¹¹, R¹² und R¹³ sind unabhängig gewählt aus der Gruppe bestehend aus Alkyl, Alkenyl, Cycloalkyl, Cycloalkenyl, Alkoxy, Aryl, Arylalkyl, Heterozyklus, Heterozyklus(alkyl) und Cyano;
R¹⁴ ist gewählt aus der Gruppe bestehend aus Wasserstoff, Alkyl, Alkenyl, Cycloalkyl, Cycloalkenyl, Alkoxy, Aryl, Arylalkyl, Heterozyklus, Heterozyklus(alkyl) und Cyano;
X² ist gewählt aus der Gruppe bestehend aus -O-(CH₂)ₙ- und - S-(CH₂)ₙ-, worin n 0, 1 oder 2 ist;
X³ ist abwesend oder gewählt aus der Gruppe bestehend aus - CH₂- und -C(R¹⁵)(R¹⁶)-, worin R¹⁵ und R¹⁶ unabhängig gewählt sind aus der Gruppe bestehend aus Wasserstoff und Alkyl;
R⁵ und R⁶ sind gewählt aus der Gruppe bestehend aus Wasserstoff, Alkyl, substituiertem Alkyl, Alkenyl, Alkinyl, Aryl, Cycloalkyl, Heterzyklus, Aryl(substituiertes Alkyl) und Arylalkyl, oder R⁵ und R⁶ sind zusammengenommen mit den Atomen, an die sie gebunden sind, um einen 5- bis 7-gliedrigen Ring zu bilden, wahlweise aromatisch und wahlweise enthaltend ein oder zwei Heteroatome gewählt aus O, N und S, und wahlweise substituiert mit 1 bis 2 Gruppen gewählt aus Alkyl, Hydroxy, Halogen, Oxo, Haloalkyl, Cyano und Nitro;
vorausgesetzt daß, wenn n 2 ist, dann ist X³ abwesend und R⁵ und R⁶ zusammengenommen mit dem Atom, an welches sie gebunden sind, bilden einen Benzenring;
die gestrichelte Bindung stellt eine optionale Doppelbindung dar;
Aryl ist ein mono- oder bizyklisches carbozyklisches Ringsystem, das ein oder zwei aromatische Ringe hat, worin Aryl alleine oder als Teil einer anderen Gruppe wahlweise substituiert sein kann mit ein, zwei oder drei Substituenten gewählt aus der Gruppe bestehend aus C₁-C₆ Alkyl, Haloalkyl, Alkoxy, Thioalkoxy, Amino, Alkylamino, Dialkylamino, Hydroxy, Halo, Mercapto, Nitro, Carboxaldehyd, Carboxy,- Alkoxycarbonyl und Carboxamid, oder Aryl kann Tetrafluorphenyl oder Pentafluorphenyl sein;
Heterozyklus ist ein 3- oder 4-gliedriger Ring, enthaltend ein Heteroatom, gewählt aus Sauerstoff, Stickstoff und Schwefel, oder ein 5-, 6- oder 7-gliedriger Ring, enthaltend ein, zwei oder drei Stickstoffatome, ein Stickstoff- und ein Schwefelatom, oder ein Stickstoff- und ein Sauerstoffatom, worin der 5-gliedrige Ring 0-2 Doppelbindungen hat und die 6- und 7-gliedrigen Ringe 0-3 Doppelbindungen haben, und worin die Stickstoffatome wahlweise quaternisiert oder eine bizyklische Gruppe sein können, in der einer der obigen heterzyklischen Ringe ankondensiert ist an einen Benzenring oder Cyclohexanring oder einen anderen heterozyklischen Ring, wie oben definiert, worin Heterozyklus allein oder als Teil einer anderen Gruppe wahlweise substituiert sein kann mit ein oder zwei Substituenten unabhängig gewählt aus Hydroxy, Halo, Oxo, C₁-C₆ Alkylimino, Amino, Alkylamino, Dialkylamino, Alkoxy, Alkoxyalkoxy, Haloalkyl, Cycloalkyl, Aryl, Arylalkyl, -COOH, -SO₃H und C₁-C₆ Alkyl, oder Stickstoff-enthaltende Heterozyklen können N-geschützt sein;
Cycloalkyl ist ein aliphatisches Ringsystem, das 3 bis 10 Kohlenstoffatome und 1 bis 3 Ringe hat, worin Cycloalkyl allein oder als Teil einer anderen Gruppe wahlweise substituiert sein kann mit ein, zwei oder drei Substituenten unabhängig gewählt aus C₁-C₆ Alkyl, Haloalkyl, Alkoxy, Thioalkoxy; Amino, Alkylamino, Dialkylamino, Hydroxy, Halo, Mercapto, Nitro, Carboxaldehyd, Carboxy, Alkoxycarbonyl und Carboxamid;
Cycloalkenyl ist ein zyklischer Kohlenwasserstoff, enthaltend von 3 bis 8 Kohlenstoffatome und mindestens eine Kohlenstoff-Kohlenstoff-Doppelbindung, worin Cycloalkenyl wahlweise substituiert sein kann mit ein bis fünf Substituenten unabhängig gewählt aus Alkenyl, Alkoxy, Alkoxycarbonyl, Alkoxycarbonylalkyl, Alkyl, Alkylcarbonyloxy, Alkylcarbonyloxyalkyl, Alkinyl, -C(O)-NHR¹⁰, Dimethylaminocarbonyl, -Alkyl-C(O)-NHR¹⁰, Dimethylaminocarbonylalkyl, Arylalkoxycarbonyl, Arylalkoxycarbonylalkyl, Arylcarbonyloxy, Arylcarbonyloxyalkyl, Aryloxycarbonyl, Aryloxycarbonylalkyl, Halo, Haloalkoxy, Haloalkyl, Hydroxy, Hydroxyalkyl, Sulfamylalkyl, -NHR¹⁰ und (NHR¹⁰)Alkyl;
substituiertes Alkyl ist eine C₁-C₆ Alkylgruppe substituiert mit 2, 3 oder 4 Substituenten unabhängig gewählt aus der Gruppe bestehend aus Alkoxy, Alkoxycarbonyl, Alkylcarbonyl, Alkylcarbonyloxy, -C(O)-NHR¹⁰, Dimethylaminocarbonyl, Aryl, Arylalkoxycarbonyl, Arylcarbonyloxy, Aryloxycarbonyl, -CF₃, Cyano, Cycloalkyl, Halo, Haloalkoxy, Heterozyklus, Hydroxy, Sulfamyl, Alkylsulfonyl, Arylsulfonyl und -NHR¹⁰;
oder ein pharmazeutisch verträgliches Salz, Ester oder Prodrug davon.

2. Eine Verbindung von Anspruch 1, worin R¹ gewählt ist aus der Gruppe bestehend aus 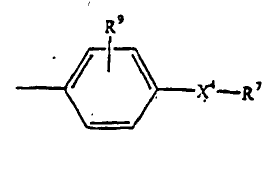 oder 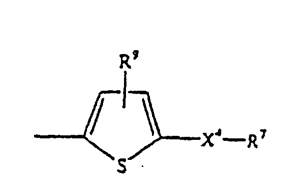 worin R⁷, X⁴ und R⁹ wie in Anspruch 1 definiert sind.

3. Eine Verbindung von Anspruch 2, worin X² Sauerstoff ist, X³ ist abwesend oder -CH₂-, und R⁵ und R⁶ bilden einen 5- bis 7-gliedrigen aromatischen oder nicht aromatischen carbozyklischen Ring, wobei der carbozyklische Ring wahlweise mono-, di- oder trisubstituiert sein kann mit Halogen.

4. Eine Verbindung von Anspruch 2, worin X² Sauerstoff ist, X³ ist abwesend oder -CH₂-, R⁵ und R⁶ sind unabhängig gewählt aus der Gruppe bestehend aus Wasserstoff, Alkyl, Cyano und Aryl.

5. Eine Verbindung von Anspruch 1, worin die Verbindung gewählt ist aus der Gruppe bestehend aus:
3-(4-Fluorphenyl)-2-(4-(methylsulfonyl)phenyl-5H-pyrazolo[1,5-*a*][3,1]benzoxazin;
3-(4-Fluorphenyl)-2-(4-(methylsulfonyl)phenyl)-5,6,7,8-tetrahydropyrazolo[5,1-*b*][1,3]benzoxazol;
3-(*Tert*-Butyl)-7-(4-fluorphenyl)-6-(4-(aminosulphonyl)phenyl)pyrazolo[5,1-*b*][1,3]oxazol;
7-(4-Fluorphenyl)-3-methyl-6-(4-(aminosulphonyl)phenyl)pyrazolo[5,1-*b*][1,3]oxazol-2-carbonitril;
3-Ethyl-7-(4-fluorphenyl)-6-(4-(methylsulfonyl)phenyl)pyrazolo[5,1-*b*][1,3]oxazol;
3-Ethyl-7-(4-fluorphenyl)-6-(4-(aminosulphonyl)phenyl)pyrazolo[5,1-*b*][1,3]oxazol;
6-Chlor-3-(4-fluorphenyl)-2-(4-(methylsulfonyl)phenyl)-5*H*pyrazolo[1,5-*a*][3,1]benzoxazin;
3-(4-Fluorphenyl)-2-(4-(aminosulfonyl)phenyl)-5*H*-pyrazolo[1,5-*a*][3,1]benzoxazin; und
2,6-Bis(4-Fluorphenyl)-3-methyl-7-(4-(methylsulphonyl)phenyl)pyrazolo[5,1-*b*][1,3]oxazol;
oder ein pharmazeutisch verträgliches Salz, Ester, Amid oder Prodrug davon.

6. Eine pharmazeutische Zusammensetzung, die eine therapeutisch wirksame Menge einer Verbindung von Anspruch 1 oder ein pharmazeutisch verträgliches Salz, Ester oder Prodrug davon und einen pharmazeutisch verträglichen Träger umfaßt.

7. Eine Verbindung von Anspruch 1 oder ein pharmazeutisch verträgliches Salz, Ester oder Prodrug davon zur Verwendung als ein therapeutischer Wirkstoff.

8. Verwendung einer Verbindung von Anspruch 1 für die Herstellung eines Medikaments zur Behandlung von Schmerzen, Fieber, Entzündung, rheumatoider Arthritis, Osteoarthritis und Krebs, durch Verabreichung einer therapeutisch wirksamen Menge an ein Säugetier, das eine solche Behandlung benötigt.

## Revendications

1. Composé ayant la formule I ci-dessous 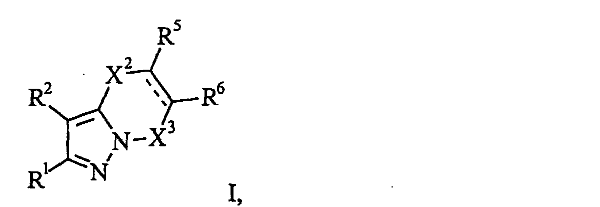 dans laquelle l'un des groupes R¹ et R² est choisi dans le groupe constitué par : 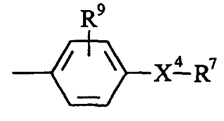 ou 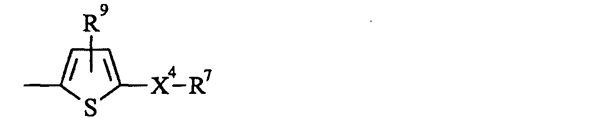 dans lesquels R⁷ est choisi dans le groupe constitué par un groupe alkyle, amino, alkylamino, dialkylamino ;
X⁴ est choisi dans le groupe constitué par -SO₂-, -SO(NR⁸)- ;
R⁸ est choisi dans le groupe constitué par un atome d'hydrogène, un groupe alkyle et cycloalkyle ;
R⁹ est choisi dans le groupe constitué par un atome d'hydrogène et un atome d'halogène ; et
l'autre groupe des groupes R¹ et R² est choisi dans le groupe constitué par un groupe hydroxyalkyle, halogène, alkyle, alcényle, alcynyle, alcoxyalkyle, - (NHR¹⁰)-C(O)-alkyle, diméthylaminocarbonylalkyle, halogénoalkyle, cycloalkyle, cycloalkylalkyle, cycloalcényle, cycloalcénylalkyle, aminocarbonylalkyle, aryle, hétérocyclique, hétérocyclo(alkyle), cyano, nitro, -Y-R¹⁰, -C(R¹¹)(R¹²)halogène et -C(R¹¹)(R¹²)OH ;
Y est choisi dans le groupe constitué par -O-, -S-, -C(R¹¹)(R¹²)-, -C(O)NR¹⁴-, - C(O)-, -C(O)O-, -NH-, -NR¹⁴C(O)- et -N(R¹³)- ;
R¹⁰ est choisi dans le groupe constitué par un atome d'hydrogène, un groupe alkyle, alcényle, alcynyle, cycloalkyle, cycloalcényle, amino, cyano, aryle, arylalkyle, hétérocyclique et hétérocyclo(alkyle) ;
R¹¹, R¹² et R¹³ sont choisis indépendamment dans le groupe constitué par un groupe alkyle, alcényle, cycloalkyle, cycloalcényle, alcoxy, aryle, arylalkyle, hétérocyclique, hétérocyclo(alkyle) et cyano ;
R¹⁴ est choisi dans le groupe constitué par un atome d'hydrogène, un groupe alkyle, alcényle, cycloalkyle, cycloalcényle, alcoxy, aryle, arylalkyle, hétérocyclique, hétérocyclo(alkyle) et cyano ;
X² est choisi dans le groupe constitué par -O-(CH₂)ₙ- et -S-(CH₂)ₙ-, dans lesquels n est 0, 1 ou 2 ;
X³ est absent ou est choisi dans le groupe constitué par -CH₂- et - C(R¹⁵)(R¹⁶)- dans lequel R¹⁵ et R¹⁶ sont choisis indépendamment dans le groupe constitué par un atome d'hydrogène et un groupe alkyle ;
R⁵ et R⁶ sont choisis dans le groupe constitué par un atome d'hydrogène, un groupe alkyle, alkyle substitué, alcényle, alcynyle, aryle, cycloalkyle, hétérocyclique, aryl(alkyle substitué) et arylalkyle ou R⁵ et R⁶ sont pris ensemble avec les atomes auxquels ils sont liés pour former un cycle à 5 à 7 chaînons, éventuellement aromatique et contenant éventuellement un ou deux hétéroatomes choisis parmi O, N et S et éventuellement substitué par 1 à 2 groupes choisis parmi un groupe alkyle, hydroxy, halogène, oxo, halogénoalkyle, cyano et nitro ;
à condition que lorsque n est égal à 2, alors X³ soit absent et R⁵ et R⁶, pris ensemble avec l'atome auquel ils sont liés, forment un cycle benzénique ;
la liaison en pointillés représente une double liaison éventuelle ;
un groupe aryle est un système cyclique carbocyclique mono- ou bicyclique comportant un ou deux cycles aromatiques, dans lequel le groupe aryle par lui-même ou en tant que partie d'un autre groupe peut être éventuellement substitué par un, deux ou trois substituants choisis dans le groupe constitué par un groupe alkyle en C₁-C₆, halogénoalkyle, alcoxy, thioalcoxy, amino, alkylamino, dialkylamino, hydroxy, halogène, mercapto, nitro, carboxaldéhyde, carboxy, alcoxycarbonyle et carboxamide ou le groupe aryle peut être un groupe tétrafluorophényle ou pentafluorophényle ;
un groupe hétérocyclique est un cycle à 3 à 4 chaînons contenant un hétéroatome choisi parmi un atome d'oxygène, d'azote et de soufre ou un cycle à 5, 6 ou 7 chaînons contenant un, deux ou trois atomes d'azote, un atome d'azote et un atome de soufre ou un atome d'azote et un atome d'oxygène, où le cycle à 5 chaînons comporte 0 à 2 doubles liaisons et les cycles à 6 et 7 chaînons comportent 0 à 3 doubles liaisons et où les atomes d'azote peuvent éventuellement être quatemisés, ou un groupe bicyclique dans lequel n'importe lequel des hétérocycles ci-dessus est condensé avec un cycle benzénique ou avec un cycle cyclohexane ou avec un autre hétérocycle tel que défini ci-dessus, où le groupe hétérocyclique par lui-même ou en tant que partie d'un autre groupe peut être éventuellement substitué par un ou deux substituants choisis indépendamment parmi un groupe hydroxy, halogène, oxo, (alkyl en C₁-C₆)imino, amino, alkylamino, dialkylamino, alcoxy, alcoxyalcoxy, halogénoalkyle, cycloalkyle, aryle, arylalkyle, -COOH, -SO₃H et alkyle en C₁-C₆ ou les hétérocycles contenant un atome d'azote peuvent être protégés sur l'azote ;
un groupe cycloalkyle est un système cyclique aliphatique comportant 3 à 10 atomes de carbone et 1 à 3 cycles, où le groupe cycloalkyle par lui-même ou en tant que partie d'un autre groupe peut être éventuellement substitué par un, deux ou trois substituants choisis indépendamment parmi un groupe alkyle en C₁-C₆, halogénoalkyle, alcoxy, thioalcoxy, amino, alkylamino, dialkylamino, hydroxy, halogène, mercapto, nitro, carboxaldéhyde, carboxy, alcoxycarbonyle et carboxamide ;
un groupe cycloalcényle est un hydrocarbure cyclique contenant de 3 à 8 atomes de carbone et au moins une double liaison carbone-carbone, où le groupe cycloalcényle peut être éventuellement substitué par un à cinq substituants choisis indépendamment parmi un groupe alcényle, alcoxy, alcoxycarbonyle, alcoxycarbonylalkyle, alkyle, alkylcarbonyloxy, alkylcarbonyloxyalkyle, alcynyle, - C(O)NHR¹⁰, diméthylaminocarbonyle, -alkyl-C(O)NR¹⁰, diméthylaminocarbonylalkyle, arylalcoxycarbonyle, arylalcoxycarbonylalkyle, arylcarbonyloxy, arylcarbonyloxyalkyle, aryloxycarbonyle, aryloxycarbonylalkyle, halogène, halogénoalcoxy, halogénoalkyle, hydroxy, hydroxyalkyle, sulfamylalkyle, -NHR¹⁰ et -(NHR¹⁰)alkyle ;
un groupe alkyle substitué est un groupe alkyle en C₁-C₆ substitué par 2, 3 ou 4 substituants choisis indépendamment dans le groupe constitué par un groupe alcoxy, alcoxycarbonyle, alkylcarbonyle, alkylcarbonyloxy, -C(O)-NHR¹⁰, diméthylaminocarbonyle, aryle, arylalcoxycarbonyle, arylcarbonyloxy, aryloxycarbonyle, - CF₃, cyano, cycloalkyle, halogène, halogénoalcoxy, hétérocyclique, hydroxy, sulfamyle, alkylsulfonyle, arylsulfonyle et -NHR¹⁰ ;
ou un de ses sels, esters ou promédicaments pharmaceutiquement acceptables.

2. Composé de la revendication 1, dans lequel R' est choisi dans le groupe constitué par 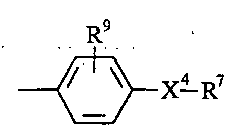 ou 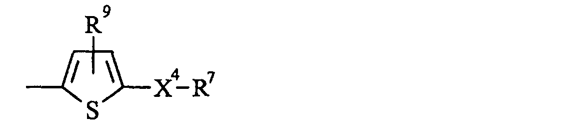 dans lesquels R⁷, X⁴ et R⁹ sont tels que définis dans la revendication 1.

3. Composé de la revendication 2, dans lequel X² est un atome d'oxygène, X³ est absent ou un groupe -CH₂- et R⁵ et R⁶ forment un cycle carbocyclique aromatique ou non aromatique à 5 à 7 chaînons, ledit cycle carbocyclique étant éventuellement mono-, di- ou trisubstitué par des atomes d'halogène.

4. Composé de la revendication 2, dans lequel X² est un atome d'oxygène, X³ est absent ou un groupe -CH₂-, R⁵ et R⁶ sont indépendamment choisis dans le groupe constitué par un atome d'hydrogène, un groupe alkyle, cyano et aryle.

5. Composé de la revendication 1, dans lequel ledit composé est choisi dans le groupe constitué par :
la 3-(4-fluorophényl)-2-(4-(méthylsulfonyl)phényl)-5H-pyrazolo[1,5-a][3,1]benzoxazine ;
le 3-(4-fluorophényl)-2-(4-(méthylsulfonyl)phényl)-5,6,7,8-tétrahydropyrazolo[5,1-b] [1,3]benzoxazole ;
le 3-(tert-butyl)-7-(4-fluorophényl)-6-(4-(aminosulfonyl)phényl)pyrazolo[5,1-b][1,3] oxazole ;
le 7-(4-fluorophényl)-3-méthyl-6-(4-(aminosulfonyl)phényl)pyrazolo[5,1-b][1,3] oxazole-2-carbonitrile ;
le 3-éthyl-7-(4-fluorophényl)-6-(4-(méthylsulfonyl)phényl)pyrazolo[5,1-b][1,3] oxazole ;
le 3-éthyl-7-(4-fluorophényl)-6-(4-(aminosulfonyl)phényl)pyrazolo[5,1-b][1,3] oxazole ;
la 6-chloro-3-(4-fluorophényl)-2-(4-(méthylsulfonyl)phényl)-5H-pyrazolo[1,5-a][3,1] benzoxazine ;
la 3-(4-fluorophényl)-2-(4-(aminosulfonyl)phényl)-5H-pyrazolo[1,5-a][3,1]benzoxazine ; et
le 2,6-bis(4-fluorophényl)-3-méthyl-7-(4-(méthylsulfonyl)phényl)pyrazolo[5,1-b] [1,3]oxazole ;
ou un de ses sels, esters, amides ou promédicaments pharmaceutiquement acceptables.

6. Composition pharmaceutique comprenant une quantité thérapeutiquement efficace d'un composé de la revendication 1 ou d'un de ses sels, esters ou promédicaments pharmaceutiquement acceptables et un excipient pharmaceutiquement acceptable.

7. Composé de la revendication 1 ou un de ses sels, esters ou promédicaments pharmaceutiquement acceptables pour une utilisation en tant qu'agent thérapeutique.

8. Utilisation d'un composé de la revendication 1 pour préparer un médicament pour traiter la douleur, la fièvre, l'inflammation, la polyarthrite rhumatoïde, l'arthrose et le cancer par administration à un mammifère ayant besoin d'un tel traitement d'une quantité thérapeutiquement efficace.
